# EUROPEAN PATENT APPLICATION

(11) **EP 4 410 986 A2**
(43) Date of publication of application: **07.08.2024**
(21) Application number: 24178098.0
(22) Date of filing: 05.07.2018
(51) Int. Cl.: C12N 15/85

(54) **CELLS, VERTEBRATES, POPULATIONS & METHODS**

(30) Priority: 07.07.2017 GB 201710984
(62) Divisional of application: 18743421.2
(71) Applicant: Kymab Limited, Cambridge Cambridgeshire CB22 3AT (GB)
(72) Inventor: LEE, E-Chiang, Cambridge, CB22 3AT (GB); WANG, Wei, Cambridge, CB22 3AT (GB); BLACKWOOD, John Kenneth, Cambridge, CB22 3AT (GB); MAGLIOZZI, Roberto, Cambridge, CB22 3AT (GB); WOOD, Andrew, Cambridge, CB22 3AT (GB)
(74) Representative: CMS Cameron McKenna Nabarro Olswang LLP

(57) **Abstract**

The invention relates to cells and non-human vertebrates for producing antibody chains (eg, truly common L or H chains), in particular for use in producing multi-specific antibodies useful for therapy or diagnosis.

## Description

### FIELD OF THE INVENTION

The invention relates to cells and non-human vertebrates for producing antibody chains, in particular for use in producing multi-specific antibodies useful for therapy or diagnosis.

### BACKGROUND

Antibodies are a rapidly growing drug class. A typical 4-chain antibody contains two identical heavy chains and two identical light chains, which form a molecule with bivalent and monspecific antigen binding features. The bispecific antibody is a new class of drug which can bind two different antigens or two different epitopes of the same antigen at the same time. There are several applications of bispecific antibodies for disease treatment. The most widely used application of this concept is in cancer immunotherapy, where bispecific antibodies engage cytotoxic T cells and target to tumour cells to be destroyed. They can also be applied to simultaneously block to two targets (Klein, C et al. MABs. 2016. 8: 1010-20.), or act as a cofactor for an enzymatic pathway (Kitazawa, T. et al. Not Med. 2012. 18: 1570-4.). Two bispecific antibodies, catumaxomab (anti-EpCAM x anti-CD3) and blinatumomab (anti-19 x anti-CD3) have been approved for therapy, and several additional bispecific antibodies are currently in clinical development. There are five different structural groups for bispecific antibodies: (i) bispecific IgG (BsIgG) (ii) IgG appended with an additional antigen-binding moiety (iii) BsAb fragments (iv) bispecific fusion proteins and (v) BsAb conjugates (Spiess, C. et al. Mol. Immunol. 2015. 67:95-106.). Among them, BsIgG is a bispecific antibody with molecular weight close to a IgG molecule. It usually has similar biological and biophysical features to a typical IgG. Production of BsIgG by co-expression of the two antibodies in cells is a highly challenge because of the low yield of desired BsIgG and the difficulty to remove closely related mispaired IgG molecules. Two heavy chains can from homodimers as well as desired heterodimers. In addition, light chains can mispair with non-cognate heavy chains. Consequently, coexpression of two antibodies results in nine unwanted IgG species and only one desired BsIgG due to the heavy chain pairing and light chain pairing problems.

Engineering heavy chains to favour the heterodimerization has been demonstrated by the "knob-into-hole" technology (Ridgway, J.B. et al. Protein Eng. 1996. 9: 617-21.; Atwell, S. et al. J. Mol. Biol. 1997. 270: 26-35.) or the "charge pairing" technology (Gunasekaran, K. et al. J. Biol. Chem. 2010. 285: 19637-46.; Strop, P. et al. J. Mol. Biol. 2012. 420: 204-19.). These technologies aim to resolve the heavy chain pairing problem. The light chain pairing problem is obviated by expression of antibodies using common light chains (Merchant, M. et al. Not. Biotechnol. 1998. 16: 677-81.). By applying the "knob-into-hole" or "charge pairing", and common light chain technologies, coexpression of one light chain and two heavy chains results in one dominant BsIgG and three minor mispairing species. The knobs-into-holes technology is described in US5,731,168 and WO98/50431. Purification steps using cation exchange (Sampei, Z. et al. PLoS One. 2013. 8: e57479.) or differential protein A binding (Smith, E.J. et al. Sci. Rep. 2015. 5: 17943.) have been applied to isolate this dominant expressed BsIgG.

It is challenging to identify a common light chain for BsIgG. Forced pairing of non-cognate heavy and light chains usually results in low yield of expression or instability of molecules. Light chain shuffling between two cognate paired light chains has been used to identify the common light chain (Sampei, Z. et al. PLoS One. 2013. 8: e57479.). This, however, relies on tedious steps and a lot of effort, and the outcome is also uncertain. Compared to this method, the "common light chain transgenic" technology is promised to be more straightforward and simple, relying on *in vivo* selection to generate the natural pairing molecules. In these applications, mice engineered to contain only one chimeric light chain-encoding sequence (human variable and mouse constant regions) are used to identify antibodies sharing the similar but not necessarily identical light chain because the inserted rearranged light chain sequence is still susceptible to somatic hypermutation (SHM). The inserted light chain choice is also limited as it relies on germline transmitted mice with one light chain sequence insertion. WO2004/106375, WO2009/1577771, EP2147594 and WO2014/160179 discuss techniques relevant to production of bispecific antibodies and common light chain mice.

### STATEMENT OF INVENTION

The inventors realised that the provision of a V domain coding variable region sequence 3' of the intronic enhancer in an antibody chain locus minimises SHM of the V region, which allows the invention to be used to identify and use true common light chains, VL domains and VH domains that can pair with these to produce antigen binding sites.

Thus, in a first configuration, the invention provides:-
A cell comprising an antibody light chain locus (first locus), wherein the locus comprises (in 5' to 3' direction)
(a) a light chain intronic enhancer;
(b) a rearranged antibody variable region encoding a rearranged antibody V domain; and
(c) an antibody light chain constant region encoding a light chain C domain;
wherein the locus is operable to express an antibody chain comprising (in N- to C-terminal direction) said rearranged V domain and said C domain.

In a second configuration, the invention provides:-
A cell comprising an antibody heavy chain locus (first locus), wherein the locus comprises (in 5' to 3' direction)
(a) a heavy chain intronic enhancer (Eµ);
(b) a rearranged antibody variable region encoding a rearranged antibody V domain;
(c) an optional switch sequence; and
(d) an antibody heavy chain constant region encoding a heavy chain CH1 domain;
wherein the locus is operable to express an antibody chain comprising (in N- to C-terminal direction) said rearranged V domain and said CH1 domain.

In a third configuration, the invention provides:-
A transgenic non-human vertebrate comprising a plurality of cells according to the invention.

In a fourth configuration, the invention provides:-
A transgenic non-human vertebrate whose antibody light chain repertoire is at least 95% pure for a single rearranged VL domain species.

In a fifth configuration, the invention provides:-
A transgenic non-human vertebrate whose antibody light chain repertoire comprises VL domains derived from the recombination of a first VL gene segment and a first JL gene segment, wherein at least 95% of all VL domains derived from said recombination comprise the same VL amino acid sequence.

In a sixth configuration, the invention provides:-
A plurality of spleen, bone marrow, B-cells or blood cells of a vertebrate according to the invention comprising a plurality of said first loci.

In a seventh configuration, the invention provides:-
A population of spleen, bone marrow, B-cells, hybridomas, CHO cells, HEK cells, MEF cells, COS cells, HeLa cells or blood cells, wherein each cell is according to the invention; and wherein
(a) at least 95% of the antibodies share the same light chain VL domain and the population comprises at least 10 different VH domain species; or
(b) the antibodies comprise VL domains derived from the recombination of a first VL gene segment and a first JL gene segment, wherein at least 95% of all said VL domains derived from said recombination comprise the same VL amino acid sequence and the population comprises at least 10 different VH domain species.

In a eighth configuration, the invention provides:-
A polyclonal antibody population, wherein at least 95% of the antibodies comprised by the population share the same light chain VL domain and the population comprises at least 10 different VH domain species.

In a ninth configuration, the invention provides:-
A polyclonal antibody population, comprising VL domains derived from the recombination of a first VL gene segment and a first JL gene segment, wherein at least 95% of all said VL domains derived from said recombination comprise the same VL amino acid sequence and the population comprises at least 10 different VH domain species.

In a tenth configuration, the invention provides:-
A method of identifying or obtaining an antibody, an antibody variable domain, a nucleotide sequence encoding an antibody or an antibody variable domain, or an expression vector or host cell that is capable of expressing the antibody or domain, wherein the antibody or domain is capable of specifically binding to a target antigen, the method comprising
(a) contacting the cell population of the invention, or the antibody population of the invention with said antigen (eg, immobilised on a solid support);
(b) binding antibodies expressed or comprised by said population to said antigen; and
(c) isolating or identifying one or more antibodies that bind to the antigen, or isolating or identifying a VH and/or a VL domain of a said one or more antibody; or identifying a nucleotide sequence encoding a said VH or VL; and
(d) optionally
   (i) correlating a said identified antibody or domain with a nucleotide sequence encoding therefor, thereby identifying said sequence;
   (ii) amplifying said sequence (eg, using PCR) and inserting the sequence into an expression vector or a host cell genome for expression of the encoded antibody or domain; and
   (iii) optionally expressing and isolating said antibody or domain (eg, wherein the domain is comprised by an antibody chain),
   wherein steps (i) and (ii) can be carried out in any order.

In a eleventh configuration, the invention provides:-
A plurality of B-cells, hybridomas, CHO cells, HEK cells, MEF cells, COS cells, HeLa cells that expresses the polyclonal antibody population of the invention.

In a twelfth configuration, the invention provides:-
A method obtaining a nucleotide sequence encoding an antibody or an antibody variable domain, wherein the antibody or domain is capable of specifically binding to a target antigen, the method comprising
(a) obtaining VL and/or VH-encoding nucleotide sequences from the cells of the invention;
(b) amplifying said sequence (eg, using PCR); and
(c) optionally inserting the sequence into an expression vector or a host cell genome for expression of the encoded antibody or domain.

In a thirteenth configuration, the invention provides:-
A method obtaining an antibody-producing cell line for the production of antibodies that specifically bind to an antigen, the method comprising inserting VH and VL-encoding nucleotide sequences into the genome of a host cell (eg, a CHO, HEK, MEF, COS or HeLa cell), wherein
(a) each VH sequence is operably inserted 5' of an antibody heavy chain constant region (eg, a human constant region) for expression of heavy chains by the cell comprising VH and C domains;
(b) each VL sequence is operably inserted 5' of an antibody light chain constant region (eg, a human constant region) for expression of light chains by the cell comprising VL and C domains;
(c) wherein the expressed heavy chains are capable of pairing with the light chains to produce heavy-light chain pairs, each pair comprising a VH/VL binding site that is capable of binding to an antigen; and
(d) wherein VH and VL-encoding nucleotide sequences are the sequences of VH and VL-encoding nucleotide sequences of one or more cells of the invention.

In a fourteenth configuration, the invention provides:-
A method of producing bi-specific 4- chain antibodies wherein the bi-specific antibodies comprise a respective binding site for first and second antigens, wherein the antigens are different, each antibody comprising a first heavy chain-light chain pair and a second heavy chain-light chain pair, wherein
(a) the heavy chain of the first heavy chain-light chain pair comprises a first VH, wherein the first VH (when expressed as part of first heavy chains) is capable of pairing with a VL (when expressed as part of light chains) to form a first VH/VL binding site that is capable of specifically binding to the first antigen; and
(b) the heavy chain of the second heavy chain-light chain pair comprises a second VH, wherein the second VH (when expressed as part of second heavy chains) is capable of pairing with said VL (when expressed as part of light chains) to form a second VH/VL binding site that is capable of specifically binding to the second antigen;
   the method comprising
(c) producing a first cell line that is capable of expressing the first heavy chains by (i) inserting into a first cell genome a nucleotide sequence encoding said first VH, wherein the VH sequence is operably inserted 5' of an antibody heavy chain constant region (eg, a human constant region) for expression of first heavy chains comprising first VH and C domains; and (ii) culturing the cell to produce a first cell line that expresses first heavy chains;
(d) producing a second cell line that is capable of expressing the second heavy chains by (i) inserting into a second cell genome a nucleotide sequence encoding said second VH, wherein the VH sequence is operably inserted 5' of an antibody heavy chain constant region (eg, a human constant region) for expression of second heavy chains comprising second VH and C domains; and (ii) culturing the cell to produce a second cell line that expresses second heavy chains;
(e) expressing first heavy chains from the first cell line and second heavy chains from the second cell line and mixing together first heavy chains comprising said first VH, second heavy chains comprising said second VH and light chains comprising said VL, whereby (iii) first heavy chains pair with light chains to produce said first heavy chain-light chain pairs, (iv) second heavy chains pair with light chains to produce said second heavy chain-light chain pairs; and (v) the first heavy chain-light chain pairs pair with the second heavy chain-light chain pairs, thereby producing said bi-specific antibodies
wherein VH and/or VL-encoding nucleotide sequences are the sequences of VH and/or VL-encoding nucleotide sequences of one or more cells of the invention.

In a fifteenth configuration, the invention provides:-
A method of producing bi-specific 4-chain antibodies wherein the bi-specific antibodies comprise a respective binding site for first and second antigens, wherein the antigens are different, each antibody comprising a first heavy chain-light chain pair and a second heavy chain-light chain pair, wherein
(a) the heavy chain of the first heavy chain-light chain pair comprises a first VH, wherein the first VH (when expressed as part of first heavy chains) is capable of pairing with a VL (when expressed as part of light chains) to form a first VH/VL binding site that is capable of specifically binding to the first antigen; and
(b) the heavy chain of the second heavy chain-light chain pair comprises a second VH, wherein the second VH (when expressed as part of second heavy chains) is capable of pairing with said VL (when expressed as part of light chains) to form a second VH/VL binding site that is capable of specifically binding to the second antigen;
   the method comprising
(c) mixing together first heavy chains comprising said first VH, second heavy chains comprising said second VH and light chains comprising said VL, whereby (i) first heavy chains pair with light chains to produce said first heavy chain-light chain pairs, (ii) second heavy chains pair with light chains to produce said second heavy chain-light chain pairs; and (iii) the first heavy chain-light chain pairs pair with the second heavy chain-light chain pairs, thereby producing said bi-specific antibodies wherein VH and/or VL are encoded by VH and/or VL-encoding nucleotide sequences, wherein the nucleotide sequences are the sequences of VH and/or VL-encoding nucleotide sequences of one or more cells of the invention.

In a sixteenth configuration, the invention provides:-
A method of producing a pharmaceutical composition for treating or preventing a disease or condition (eg, a cancer) in a human or non-human animal subject, the method comprising
(a) expressing an antibody from a cell line produced by the method of the inventon;
(b) mixing the antibody with a diluent or excipient;
(c) optionally wherein the antibody is identical to an antibody obtained by the method of the invention.

In a seventeenth configuration, the invention provides:-
A method of producing a pharmaceutical composition for treating or preventing a disease or condition (eg, a cancer) in a human or non-human animal subject, the method comprising mixing a bi-specific antibody with a diluent or excipient, wherein the antibody is identical to an antibody obtained by the method of the invention.

In a eighteenth configuration, the invention provides:-
A method of producing a cell or vertebrate of the invention, the method comprising
(a) obtaining a nucleic acid comprising a rearranged antibody variable region encoding a rearranged antibody V domain; and
(b) inserting the variable region or a copy thereof into the genome of a cell, whereby the variable region is comprised by an antibody locus in said genome,
   wherein the locus comprises (in 5' to 3' direction)
(c) an antibody locus intronic enhancer;
(d) said rearranged antibody variable region; and
(e) an antibody constant region encoding an antibody C domain;
   wherein the locus is operable to express an antibody chain comprising (in N- to C-terminal direction) said rearranged V domain and said C domain; and
(f) optionally, wherein the cell is a non-human vertebrate ES cell or an iPS cell, the method further comprising generating the non-human vertebrate of the invenetion from the cell.

In a ninteenth configuration, the invention provides:-
A non-human vertebrate (eg, rodent, rat or mouse) blastocyst or pre-morula embryo implanted with an ES or iPS cell according to the invention or obtained according to the method of the invention.

In a twentieth configuration, the invention provides:-
A nucleic acid comprising a transgene for use in the method of the invention, the transgene comprising (in 5' to 3' direction)
(a) said intronic enhancer;
(b) said rearranged antibody variable region; and
(c) said antibody constant region.

In a twenty-first configuration, the invention provides:-
A nucleic acid comprising a rearranged variable region sequence encoding a rearranged V domain for use in the method of the invention, the nucleic acid comprising said rearranged variable region sequence flanked
(a) 5' by a homology arm for hybridising to a first nucleotide sequence of the cell genome, and/or
(b) 3' by a homology arm for hybridising to a second nucleotide sequence of the cell genome; Wherein
(c) the first nucleotide sequence is homologous to a sequence of an antibody light chain (eg, kappa) locus of the cell 5' of the CL region thereof, and the second nucleotide sequence is homologous to a sequence of said locus 3' of the intronic enhancer (eg, Eiκ) of the locus, whereby homologous recombination between the homology arms and the genome of the cell is capable of inserting the variable region sequence, thereby producing a locus comprising (in 5 to 3' direction) an intronic enhancer, said variable region and a CL region; or
(d) the first nucleotide sequence is homologous to a sequence of a heavy chain antibody locus of the cell 5' of the Cµ region thereof, and the second nucleotide sequence is homologous to a sequence of said locus 3' of the intronic enhancer (Eµ) of the locus, whereby homologous recombination between the homology arms and the genome of the cell is capable of inserting the variable region sequence, thereby producing a locus comprising (in 5 to 3' direction) an intronic enhancer, said variable region and a Cµ region.

Further exemplification is provided below by way or worked experiments and data.

### BRIEF DESCRIPTION OF THE DRAWINGS

**Figure 1**
   A schematic of the targeting strategy of generating a common light chain allele. The mouse κ intronic enhancer and mouse κ constant region (4.6 kb) in mouse ES cells were replaced by a synthesised DNA fragment of 6.8 kb, which contains a EF1α-Puro-2A-EGFP cassette, the natural promoter and signal peptide of human Vλ 3-21, mouse κ intronic enhancer, as well as a recombined human common lambda light chain allele from an antibody that specifically binds Target X.
**Figure 2**
   A schematic of the sorting strategy to isolate spleen B cells that express antibodies with a human λ light chain and specifically bind human Target X from a common light chain knock-in chimera mouse. The sorting strategy involved lymphocyte section, followed by single cell selection, followed by live cell selection, followed by CD19⁺ B-cell selection and finally human lambda positive and antigen (Target X) positive cell selection. SSC: side scatter. FSC: forward scatter. SSC-A: side scatter area.
**Figure 3**
   Sorting plots showing the isolation of B cells that express antibodies with a human λ light chain and specifically bind human Target X from a common light chain knock-in chimera mouse. Graphs A-F are sorting plots and show the flow of sorting. The cells selected in the first sort were used for the subsequent sort and the selection process continued. A. Sorting plot selecting lymphocytes. SSC-A (side scatter area) versus FSC-A (forward scatter area). B. Sorting plot selecting single cells. FSC-W (forward scatter width) versus FSC-A (forward scatter area). C. Sorting plot selecting single cells. SSC-W (side scatter width) versus SSC-A (side scatter area). D. Sorting plot selecting live cells. SSC-A (side scatter area) versus live/dead 7-AAD. E. Sorting plot selecting CD19⁺ B-cells. Y-axis indicates markers used versus CD19-PB. F. Sorting plot of B-cells that express human lambda light chain and specifically bind human Target X. Human Target X antigen labelled with AF647 versus human lambda light chain labelled with PE.
**Figure 4**
   VL regions of the light chains recovered by NGS essentially all carry the same 6 non-germline mutations as the knock-in common light chain allele.
**Figure 5**
   Chimera mice generated from Rag^{-/-} blastocysts micro-injected with targeted ES cells (targeted Rag^{-/-}) had similar total viable spleen cell number compared to wild-type mice (Wild-Type).
**Figure 6**
   Chimera mice generated from Rag^{-/-} blastocysts micro-injected with targeted ES cells (targeted Rag^{-/-}) had similar CD19-postive, B220-positve B-cell number compared to wild-type mice (Wild-Type).
**Figure 7**
   Percentage CD19-positive, B220-positive splenic B-cell population in chimera mice generated from Rag^{-/-} blastocysts micro-injected with targeted ES cells (targeted Rag^{-/-}) was similar to wild-type mice (Wild-Type).
**Figure 8**
   Percentage CD19-positive, B220-positive splenic B-cell population in chimera mice generated from Rag^{-/-} blastocysts micro-injected with targeted ES cells (targeted Rag^{-/-}) was similar to wild-type mice (Wild-Type).
**Figure 9**
   Percentage antigen positive IgG B-cell population in chimera mice generated from Rag^{-/-} blastocysts micro-injected with targeted ES cells (targeted Rag^{-/-}) was similar to wild-type mice (Wild-Type).

### DETAILED DESCRIPTION

Aspects herein may be expressed in terms of the non-human vertebrate being a mouse, but it is to be understood that rodents, rats, rabbits or any other non-human vertebrate may be suitable for performing the present invention.

The inventors realised that the provision in an antibody light or heavy chain locus of a V domain coding variable region sequence 3' of the intronic enhancer in an antibody chain locus minimises SHM of the V region, which allows the invention to be used to identify true common light or heavy chains. In some embodiments, the locus is a modified version of an endogenous antibody locus of a wild-type or transgenic vertebrate cell or non-human vertebrate. In other embodiments, the locus is not at an endogenous antibody chain locus, but instead is comprised by a locus inserted at Rosa 26 or another location where the locus is capable of expressing antibody chains. For example, the locus of the invention is comprised by a randomy inserted transgene in the cell or vertebrate genome. By minimising or eliminating SHM of the V region, a single (ie, "common") light chain is more likely to be expressed by the cell or such cells comprised by the vertebrate. When SHM is eliminated the variable region expresses only a single type of VL domain which may pair with different VH domains expressed by the vertebrate, thereby allowing screening and identification of one or more VH domains that may functionally pair with the VL to form VH/VL binding sites for an antigen or epitope. Even if some mutation may occur, the invention restricts the possibility of this so that the purity of VL domains produced by the same V-J recombination is likely to be such that a single rearranged VL by far predominates (eg, at a level of at least 95% or 100% as described further below). Usefully, therefore, the expression of a desired common light chain can be pre-determined (eg, by providing a known variable region (or light chain) sequence in the locus or transgene, such as a known rearranged variable region (or light chain) sequence encoding a VL (or light chain) of a known antibody that can specifically bind to a predetermined antigen or epitope. Advantageously, the ability in embodiments to use a light chain sequence (eg, a human rearranged VκJκCκ of a predetermined human antibody of known binding specificity and/or affinity for an antigen or epitope) is useful as pairings and selection *in vivo* in the vertebrate of the invention is then possible in the context of the complete human light chain that will be used in a final antibody product (eg, a bispecific antibody medicament).

Optionally, the common light chain comprises or consists of the amino acid sequence of SEQ ID NO: 1, or an amino acid sequence that is at least 70, 75, 80, 85, 90, 95, 96, 97, 98 or 99% identical thereto. Optionally, the variable domain of the common light chain comprises or consists of the variable domain of the light chain sequence of SEQ ID NO: 1, optionally with 5 or less, 4 or less, 3 or less, 2 or 1 amino acid change. Each change can be an amino acid deletion, substitution or addition. In an example, the sequence of the variable domain is determined by Kabat numbering. In an example, the sequence of the variable domain is determined by IMGT numbering.

An example of an *in vivo* approach using the chimaeric mouse (or other no-human vertebrate) to identify common light chains for muti- or bi-specific antibodies is provided, wherein B cells from the mouse are derived from ES cells with rearranged light chain loci and expressing an immunoglobulin light chain repertoire with a single or highly limited number of light chain variable domains. The chimeric mouse is, for example, generated from wild type, RAG knock-out or IgH knock-out mouse blastocysts injected with mouse ES cells carrying the knock-in rearranged human light chain encoding sequence and an unrearranged human or endogenous IgH locus. The DNA sequence of the rearranged human light chain is inserted such that it may be free of somatic hypermutation (SHM). Methods for making such a chimaeric mouse or other non-human vertebrate expressing common light chains are provided. Methods for identify antigen-specific common light chain antibodies useful as bispecific antibodies (eg, for cancer therapy in a human) are provided, as are such antibodies *per se* and cells expressing these.

A particular example work flow may be:-
1. Obtaining a first antibody comprising first human VH and VL domains, wherein the antibody specifically binds to a first predetermined antigen; or obtaining nucleotide sequences encoding said VL or the light chain of the antibody;
2. Obtaining a transgenic mouse ES cell comprising an IgH locus (in heterozygous or homozygous form), wherein the IgH locus comprises a humanised variable region for expressing a plurality of different human VH domains;
3. Inserting a nucleotide sequence encoding the light chain of the antibody or the VL thereof into the genome of the ES cell, wherein the insertion is between the Eiκ and Cκ of a kappa locus allele of the ES cell genome;
4. Optinally modifying the other kappa locus allele so that it is not capable of expressing kappa light chains, or modifying the allele whereby a nucleotide sequence encoding the light chain of the first or a second antibody or the VL thereof is inserted between the Eiκ and Cκ of said other kappa locus allele;
5. Developing the ES cell into a mouse (eg, by implanting the ES cell into a recipient blastocyst or pre-morula embryo (eg, a RAG and/or IgH knock-out blastocyst or pre-morula embryo) and implanting the blastocyst or pre-morula embryo into a pseudopregnant female mouse, whereby one or more pups are birthed from the female), wherein the mouse or pup(s) so developed comprises at least one kappa locus that is capable of expressing the light chain or VL, wherein the light chain or VL pair with heavy chains or VH expressed by the mouse to form VH/VL antigen binding sites;
6. Optionally obtaining progeny mice, wherein the progeny each comprise at least one kappa locus that is capable of expressing the light chain or VL, wherein the light chain or VL pair with heavy chains or VH expressed by the progeny mouse to form VH/VL antigen binding sites;
7. Immunising the mouse of step 5 or a said progeny mouse with a second antigen, wherein the first and second antigens are different;
8. Obtaining from an immunised mouse of step 7 (eg, using PCR of DNA of a B-cell and/or hybridoma technology of a B-cell of an immunised mouse of step 7) a nucleotide sequence encoding a VH (second VH) that pairs with the VL (or a VH of a heavy chain that pairs with the VL of the light chain), wherein the second VH and VL form an antigen binding site that specifically binds to the second antigen;
9. Optionally inserting a nucleotide sequence encoding the first VH, a nucleotide sequence encoding the second VH, and a nucleotide sequence encoding the VL into one or more expression vectors or into the genome of one or more host cells, each V nucleotide sequence being in operable connection 5' of an antibody first CH, second CH or CL region respectively for expression of first antibody heavy chains, second antibody heavy chains or antibody light chains respectively; optionally wherein the first and second CH regions encode first and second CH domains that are mutated human CH domains that pair together (eg, using charge pair mutation or knob-in-hole mutation) and/or wherein the first and second chains have different pl (eg, separated by 0.5-1 pl unit);
10. Optionally obtaining host cells of step 9 and expressing the heavy and light chains in the host cells, whereby bi-specific antibodies are produced each comprising a first heavy chain/light chain pair comprising a VH/VL binding site that is capable of specifically binding to the first antigen, and a second heavy chain/light chain pair comprising a VH/VL binding site that is capable of specifically binding to the second antigen; and
11. Optionally isolating said bi-specific antibodies.

In an alternative, first and second eptiopes of an antigen are used instead of first and second antigens. In an embodiment, the method comprises steps 1 to 8; 1 to 9; 1 to 10 or 1 to 11. In an example, there is provided a method for obtaining a bispecific antibody, the method commencing at step 7. In an example, there is provided a method for obtaining a bispecific antibody, the method commencing at step 8. In an example, there is provided a method for obtaining a bispecific antibody, the method commencing at step 9. In an example, there is provided a method for obtaining a bispecific antibody, the method commencing at step 10. In an example, there is provided a method for obtaining a bispecific antibody, the method commencing at step 11.

In an example, the light chain repertoire of each mouse used in step 7 is at least 95, 96, 97, 98 or 99% pure (or 100% pure) for the VL. This can be determined, for example, by obtaining a B-cell sample (eg, spleen and/or bone marrow B-cell sample) from the mouse, obtaining VL-encoding nucleotide sequences thereof (eg, mRNA or cDNA) and carrying out next-generation sequencing (NGS) to determine the percentage of the VL (and any other VL species) in the sampled nucleotide sequences. The skilled person will be familiar with routine NGS techniques. Thus, for example, herein "95% pure" in respect of the VL means that the repertoire (eg, a sampled repertoire of the mouse) comprises sequences (eg, mRNA, cDNA or DNA) encoding said predetermined VL in a proportion in the repertoire or sample of 95% or higher, eg, as determined by NGS. In an embodiment, all of (or substantially all of) the VL domains expressed by the mouse are identical to the VL of the antibody of step 1. This may be advantageously be achieved due to the positioning according to the invention of the VL-encoding sequence 3' of the intronic enhancer, such as between this enhancer and the constant region.

In an alternative, the VL-encoding variable region sequence (eg, in steps 3 and 4 above) is an insert in the lambda locus allele(s) of the cell or mouse between mouse lambda 2-4 and Cλ or between mouse lambda 4-10 enhancer and Cλ. Additionally, in an embodiment the VL-encoding variable region sequence is an insert in the kappa locus allele(s) of the cell or mouse between Eiκ and Cκ.

In an alternative, only one of the lambda locus alleles comprises a said insert; and only one of the kappa locus alleles comprises a said insert.

The VL-encoding variable region inserts in the lambda and kappa, or in the 2 lambda or in the 2 kappa alleles may be the same or different (ie, encode the same or different VLs, such as VLs of 2 different antibodies of predetermined specificity for the same or different antigens).

In an embodiment, first and second mouse (or other non-human vertebrate, such as rat) ES cells are modified to comprise first and second loci of the invention, eg, each cell being modified according to steps 1 to 4, but wherein the VL-encoding nucleotide sequence inserts in the cells are different (ie, encode different VL domains, such as VL domains of different antibodies of different antigen or epitope specificity). The first and second ES cells (or progeny thereof) are implanted into the same blastocyst or pre-morula embryo and a mouse (or progeny thereof) developed, wherein the mouse or progeny express said different VL domains for pairing with VH domains (eg, human VH) expressed by the mouse or progeny.

Advantageously, a first generation mouse (ie, grown directly from the ES cell-implanted blastocyst or pre-morula herein) is useful as it can be immunised without the need for further breeding to subsequent generation mice. This saves time and complexity of breeding. It may be desirable, for example, for the blastocyst or pre-morula that receives the ES cell implant to comprise a knock-out of its IgH loci or be a RAG knock-out, as this forces all productive B-cells produced in the first generation mouse (or subsequent generation mice) to be ES cell derived, thereby ensuring that all productive B-cells comprise one or more loci of the invention.

Optionally, thus generally it may advantageously not be necessary to obtain germline transmission of the loci of the invention in non-human vertebrate progeny or lineages, instead it is possible to work with chimaera vertebrates grown from the blastocyst or pre-morula embryo which provides significant time advantages over prior art techniques where germline transmission of light chain allele modifications is described. Thus, in one embodiment, a mouse or vertebrate of the invention is a chimeaera comprising a first plurality of cells of the invention and a second plurality of cells that are not according to the invention (eg, wild-type or transgenic cells that do not comprise a locus of the invention). Optionally, the chimaera may comprise a third plurality of cells according to the invnetion comprising loci according to the invention but that are capable of expressing V domains that are different from the V domains expressed by the loci of the invention of the first plurality of cells. Thus, it is possible to develop a chimaeric vertebrate that can express first and second VL domains that are different but which are predetermined as they are expressed from loci of the invention. It may be advantageous to knock out expression of one or more light and/or heavy chain loci of the second plurality of cells so that heavy and light chains in the vertebrate are only contributed by the first and optional third plurality of cells. In addition or alternatively, the second plurality of cells may have a RAG (eg, RAG-1 and/or RAG-2) knock-out to eliminate antibody chain contribution from such cells in the vertebrate.

In an alternative configuration, it is recognised that such generation and use of chimaeras and first generation non-human vertebrates of the invention is advantageous as so described due to the lack of need for breeding and time saving, and this is not dependent upon the feature of the invention directed to providing the variable region insert 3' of the intronic enhancer. Thus, an alternative configuration is provided wherein any feature herein is *mutatis mutandis* applicable with the alternative that the locus of the invention comprises said variable region 5' of the intronic enhancer, whereby the locus is capable of expressing an antibody chain comprising the V and C domains. Furthermore, in the alternative configuration, the variable region may be an unrearranged variable region comprising a plurality of VH gene segments, one or more D gene segments and one or more JH gene segments, wherein the variable region is capable of rearranging for the expression of a plurality of different VH domains; or the variable region may be an unrearranged variable region comprising a plurality of VL gene segments and one or more JH gene segments, wherein the variable region is capable of rearranging for the expression of a plurality of different VL domains; or only one two, three, four or five rearranged variable regions may be 5' of the enhancer for expressing a restricted diversity of V domains (eg, VL domains). Particularly, this configuration is useful wherein the vertebrate is a chimaera, such a first generation vertebrate as described herein. This alternative configuration of the invention also provides for a method of immunising such a chimaera or vertebrate with an antigen and isolating an antibody that specifically binds to the antigen, a VH domain thereof, a VL domain thereof, or a nucleotid sequence encoding any of these. Beneficially, this method is streamlined by the possibility of immunising a vertebrate that is produced without need for breeding cycles.

A V domain or binding site that "specifically binds to" or is "specific for" a particular antigen or epitope is one that binds to that particular antigen or epitope without substantially binding to other antigens or epitopes. For example, binding to the antigen or epitope is specific when the antibody binds with a K_{D} of 1mM or less, eg, 100 µM or less, 10 µM or less, 1 µM or less, 100 nM or less, eg, 10 nM or less, 1 nM or less, 500 pM or less, 100 pM or less, or 10pM or less. The binding affinity (K_{D}) can be determined using standard procedures as will be known by the skilled person, eg, binding in ELISA and/or affinity determination using surface plasmon resonance (eg, Biacore^{™}, Proteon^{™} or KinExA^{™} solution phase affinity measurement which can detect down to fM affinities (Sapidyne Instruments, Idaho)). In one embodiment, the surface plasmon resonance (SPR) is carried out at 25°C. In another embodiment, the SPR is carried out at 37°C. In one embodiment, the SPR is carried out at physiological pH, such as about pH7 or at pH7.6 (eg, using Hepes buffered saline at pH7.6 (also referred to as HBS-EP)). In one embodiment, the SPR is carried out at a physiological salt level, eg, 150mM NaCl. In one embodiment, the SPR is carried out at a detergent level of no greater than 0.05% by volume, eg, in the presence of P20 (polysorbate 20; eg, Tween-20TM) at 0.05% and EDTA at 3mM. In one example, the SPR is carried out at 25°C or 37°C in a buffer at pH7.6, 150mM NaCl, 0.05% detergent (eg, P20) and 3mM EDTA. The buffer can contain 10mM Hepes. In one example, the SPR is carried out at 25oC or 37oC in HBS-EP. HBS-EP is available from Teknova Inc (California; catalogue number H8022).

In an example, the affinity is determined using SPR by
1. Coupling anti-mouse (or other relevant non-human vertebrate, to match the C region of an antibody for example) IgG (eg, Biacore BR-1008-38) to a biosensor chip (eg, GLM chip) such as by primary amine coupling;
2. Exposing the anti-mouse IgG (non-human vertebrate antibody) to a test IgG antibody or heavy chain to capture test antibody on the chip;
3. Passing the test antigen over the chip's capture surface at 1024nM, 256nM, 64nM, 16nM, 4nM with a 0nM (i.e. buffer alone); and
4. And determining the affinity of binding of test antibody/chain to test antigen using surface plasmon resonance, eg, under an SPR condition discussed above (eg, at 25oC in physiological buffer). SPR can be carried out using any standard SPR apparatus, such as by BiacoreTM or using the ProteOn XPR36TM (Bio-Rad^{®}).

Regeneration of the capture surface can be carried out with 10mM glycine at pH1.7. This removes the captured antibody and allows the surface to be used for another interaction. The binding data can be fitted to 1:1 model inherent using standard techniques, eg, using a model inherent to the ProteOn XPR36TM analysis software.

The term "antibody" includes monoclonal antibodies (including full length antibodies which have an immunoglobulin Fc region), antibody compositions with polyepitopic specificity, multispecific antibodies (e.g., bispecific antibodies, diabodies, and single-chain molecules, as well as antibody fragments (e.g., Fab, F(ab')2) having paired VH/VL antigen binding site(s).

A "domain" is a folded protein structure which has tertiary structure independent of the rest of the protein. Generally, domains are responsible for discrete functional properties of proteins and in many cases may be added, removed or transferred to other proteins without loss of function of the remainder of the protein and/or of the domain.

Optionally, any multispecific antibody herein is a bispecific antibody. This includes formats such as DVD-Ig, mAb², FIT-Ig, mAb-dAb, dock and lock, Fab-arm exchange, SEEDbody, Triomab, LUZ-Y, Fcab, κλ-body, orthogonal Fab, scDiabody-Fc, diabody-Fc, tandem scFv-Fc, Fab-scFv-Fc, Fab-scFv, intrabody, BiTE, diabody, DART, TandAb, scDiabody, scDiabody-CH3, Diabody-CH3, Triple body, Miniantibody, minibody, TriBi minibody, scFv-CH3 KIH, scFv-CH-CL-scFv, F(ab')2-scFv, scFv-KIH, Fab-scFv-Fc, tetravalent HCab, ImmTAC, knobs-in-holes, knobs-in-holes with common light chain, knobs-in-holes with common light chain and charge pairs, charge pairs, charge pairs with common light chain, DT-IgG, DutaMab, IgG(H)-scFv, scFv-(H)IgG, IgG(L)-scFv, scFv-(L)IgG, IgG(L,H)-Fv, IgG(H)-V, V(H)-IgG, IgG(L)-V, V(L)-IgG, KIH IgG-scFab, 2scFv-lgG, IgG-2scFv, scFv4-Ig and zybody. For a review of bispecific formats, see Spiess, C., et al.,. Mol. Immunol. (2015). Optionally, the multispecific ligand is a DVD-Ig, mAb², FIT-Ig, mAb-dAb, dock and lock, SEEDbody, scDiabody-Fc, diabody-Fc, tandem scFv-Fc, Fab-scFv-Fc, Fab-scFv, intrabody, BiTE, diabody, DART, TandAb, scDiabody, scDiabody-CH₃, Diabody-CH₃, minibody, knobs-in-holes ligand, knobs-in-holes ligand with common light chain, knobs-in-holes ligand with common light chain and charge pairs, charge pairs, ligand having charge pairs with common light chain; in each case comprising at least one VH/VL antigen binding site.

Optionally, the rearranged variable region (eg, a rearranged variable region encoding a VL, Vλ or Vκ domain, eg, a human domain) in the light chain locus comprises upstream one of the following pairs of promoter and signal peptide (ie, promoter, SP and V region in 5' to 3' order):-
1) Mouse Vλ promoter + Mouse Vλ SP
2) Mouse Vλ promoter + Non-mouse rodent Vλ SP
3) Mouse Vλ promoter + Human Vλ SP
4) Mouse Vλ promoter + Non-human primate Vλ SP
5) Mouse VK promoter + Mouse Vλ SP
6) Mouse VK promoter + Non-mouse rodent Vλ SP
7) Mouse VK promoter + Human Vλ SP
8) Mouse VK promoter + Non-human primate Vλ SP
9) Non-mouse rodent Vλ promoter + Mouse Vλ SP
10) Non-mouse rodent Vλ promoter + Non-mouse rodent Vλ SP
11) Non-mouse rodent Vλ promoter + Human Vλ SP
12) Non-mouse rodent Vλ promoter + Non-human primate Vλ SP
13) Non-mouse rodent VK promoter + Mouse Vλ SP
14) Non-mouse rodent VK promoter + Non-mouse rodent Vλ SP
15) Non-mouse rodent VK promoter + Human Vλ SP
16) Non-mouse rodent VK promoter + Non-human primate Vλ SP
17) Human Vλ promoter + Mouse Vλ SP
18) Human Vλ promoter + Non-mouse rodent Vλ SP
19) Human Vλ promoter + Human Vλ SP
20) Human Vλ promoter + Non-human primate Vλ SP
21) Human VK promoter + Mouse Vλ SP
22) Human VK promoter + non-mouse rodent Vλ SP
23) Human VK promoter + Human Vλ SP
24) Human VK promoter + Non-human primate Vλ SP
25) Non-human primate Vλ promoter + Mouse Vλ SP
26) Non-human primate Vλ promoter + Non-mouse rodent Vλ SP
27) Non-human primate Vλ promoter + Human Vλ SP
28) Non-human primate Vλ promoter + Non-human primate Vλ SP
29) Non-human primate VK promoter + Mouse Vλ SP
30) Non-human primate VK promoter + Non-mouse rodent Vλ SP
31) Non-human primate VK promoter + Human Vλ SP
32) Non-human primate VK promoter + Non-human primate Vλ SP
33) Mouse Vλ promoter + Mouse VK SP
34) Mouse Vλ promoter + Non-mouse rodent VK SP
35) Mouse Vλ promoter + Human VK SP
36) Mouse Vλ promoter + Non-human primate VK SP
37) Mouse VK promoter + Mouse VK SP
38) Mouse VK promoter + Non-mouse rodent VK SP
39) Mouse VK promoter + Human VK SP
40) Mouse VK promoter + Non-human primate VK SP
41) Non-mouse rodent Vλ promoter + Mouse VK SP
42) Non-mouse rodent Vλ promoter + Non-mouse rodent VK SP
43) Non-mouse rodent Vλ promoter + Human VK SP
44) Non-mouse rodent Vλ promoter + Non-human primate VK SP
45) Non-mouse rodent VK promoter + Mouse VK SP
46) Non-mouse rodent VK promoter + Non-mouse rodent VK SP
47) Non-mouse rodent VK promoter + Human VK SP
48) Non-mouse rodent VK promoter + Non-human primate VK SP
49) Human Vλ promoter + Mouse VK SP
50) Human Vλ promoter + Non-mouse rodent VK SP
51) Human Vλ promoter + Human VK SP
52) Human Vλ promoter + Non-human primate VK SP
53) Human VK promoter + Mouse VK SP
54) Human VK promoter + non-mouse rodent VK SP
55) Human VK promoter + Human VK SP
56) Human VK promoter + Non-human primate VK SP
57) Non-human primate Vλ promoter + Mouse VK SP
58) Non-human primate Vλ promoter + Non-mouse rodent VK SP
59) Non-human primate Vλ promoter + Human VK SP
60) Non-human primate Vλ promoter + Non-human primate VK SP
61) Non-human primate VK promoter + Mouse VK SP
62) Non-human primate VK promoter + Non-mouse rodent VK SP
63) Non-human primate VK promoter + Human VK SP
64) Non-human primate VK promoter + Non-human primate VK SP

Example rodents are rat, beaver, gopher, hamster, gerbil, porcupine, chinchilla, lemming or vole. Example non-human primates are chimpanzee, ape, gorilla, orangutan, simian, monkey (eg, rhesus macaque or cynonmolgus monkey), lemur or baboon. Sources of sequences for the promoter and/or SP include, for example, ww.imgt.org, www.ensembl.org and sequences obtained using a sample (eg, blood sample and PCR of the sequence) from such a rodent or primate or human, as will be readily apparent to the skilled addressee.

In an example, the light chain locus of the cell, vertebrate, mammal or mouse of the invention is devoid of a selection marker. In the exaples below, a selection marker was retained and the locus performed desirably. In an alternative, an excisionable selection marker can be included when constructing the locus, followed by excsission of the marker before developing the vertebrate or mouse. In an example the marker is flanked by piggyBac terminal repeats and PBase is transiently expressed in the ES cell in which the locus has been constructed, thereby excising the marker. In an example, the marker compriss puro-delta-tk flanked by piggyBac terminal repeats.

The invention can be illustrated by the following Statements.

### STATEMENTS:

1. A cell comprising an antibody light chain locus (first locus), wherein the locus comprises (in 5' to 3' direction)
   (a) a light chain intronic enhancer;
   (b) a rearranged antibody variable region encoding a rearranged antibody V domain (eg, a VH, Vλ or Vκ, eg, a VL); and
   (c) an antibody light chain constant region encoding a light chain C domain;
   wherein the locus is operable to express an antibody chain comprising (in N- to C-terminal direction) said rearranged V domain and said C domain.
   For example, the V domain and C domain are human. For example, the V domain and C domain are a human Vκ and a human Cκ, eg, as comprised by a known antibody that binds a predetermined antigen (eg, the first antigen mentioned above). For example, the V domain and C domain are a human Vλ and a human Cλ, eg, as comprised by a known antibody that binds a predetermined antigen (eg, the first antigen mentioned above). For example, the cell is a rodent, rat our mouse cell.
      Optionally, the variable domain comprises or consists of the variable domain of the light chain sequence of SEQ ID NO: 1, optionally with 5 or less, 4 or less, 3 or less, 2 or 1 amino acid change. Each change can be an amino acid deletion, substitution or addition. In an example, the sequence of the variable domain is determined by Kabat numbering. In an example, the sequence of the variable domain is determined by IMGT numbering. Optionally, the variable domain specifically binds to a protein antigen, eg, a human protein antigen.
   For example, the cell is a rodent, rat our mouse cell and the V domain and C domain are a human Vκ and a mouse Cκ, eg, as comprised by a known antibody that binds a predetermined antigen (eg, the first antigen mentioned above); or the V domain and C domain are a human Vλ and a mouse Cλ, eg, as comprised by a known antibody that binds a predetermined antigen (eg, the first antigen mentioned above).
   In an example, the constant region encodes a Cκ or a Cλ and there is no further C domain-encoding nucleotide sequence 3' of said constant region.
   Preferably, the V domain is capable of specifically binding an antigen, eg, a human antigen, such as when comprised by a VH/VL binding site of an antibody.
   Optionally, a cell of the invention is a vertebrate, mammalian, non-human mammalian, rodent, mouse, rat or human cell. Optionally, the cell is a human or rodent (eg, a mouse or a rat) cell. Optionally, the cell or mouse is a 129 or C57BL/6 strain cell or mouse (eg, a hybrid 129 or hybrid C57BL/6 strain cell or mouse). Optionally, the cell or mouse is a hybrid 129/C57BL/6 strain cell or mouse, such as a F1H4 strain cell or mouse.
   Optionally, the locus is comprised by a transgene randomly inserted in the cell genome, or at a position outside an endogenous antibody locus of the cell. Optionally, the locus is at an endogenous antibody locus of the cell. Optionally, the locus is immediately adjacent to an endogenous antibody locus of the cell, or within 10, 5, 1 or 0.5kb of an endogenous antibody locu of the cell, such as 3' or 5' thereof. Optionally, the enhancer and/or constant region is an endogenous antibody locus enhancer or constant region of the cell. Optionally, the constant region is 5' of (eg, immediately 5' of or within 500, 400, 300, 200, 100 or 50bp 5' of) an endogenous antibody locus constant region of the cell. For example, the constant region (eg, a human Cκ or Cλ) of the locus of the invention is immediately 5' of or within 500, 400, 300, 200, 100 or 50bp 5' of an endogenous Cκ constant region of the cell.
   Optionally, in any cell of the invention, the antibody variable region is no more than 400, 300, 200, 150, 100, 50, 30, 20, 10 or 5bp 3' (or 5' in the alternative configuration) of the enhancer of the locus.
   Optionally, in any cell of the invention, the antibody variable region is no more than 400, 300, 200, 150, 100, 50, 30, 20, 10 or 5bp 5' of the constant region of the locus.
   In an alternative configuration, the antibody variable region is 3' of the 3' enhancer of an endogenous antibody (eg, kappa or lambda) locus of the cell (eg, wherein the cell is a rodent, mouse or rat cell). Optionally in this configuration, the antibody variable region is no more than 400, 300, 200, 150, 100, 50, 30, 20, 10 or 5bp 3' of the 3' enhancer.
   In an example, the constant region of the locus of the invention is an endogenous antibody constant region of the cell (eg, wherein the cell is a rodent, mouse or rat cell). In another example, the constant region of the locus of the invention is a human antibody constant region (eg, wherein the cell is a rodent, mouse or rat cel), optionally wherein the human constant region is 5' of an endogenous antibody constant region of the cell (eg, an endogenous Cκ or Cλ).
   For example, the locus comprises (in 5' to 3' direction)
      (a) a light chain intronic enhancer;
      (b) a rearranged antibody human variable region encoding a rearranged antibody V domain (eg, a human V domain);
      (c) a human or mouse or rat antibody light chain constant region encoding a light chain C domain;
      wherein the locus is operable to express an antibody chain comprising (in N- to C-terminal direction) said rearranged V domain and said C domain.
   For example, the locus comprises (in 5' to 3' direction)
      (a) a light chain intronic enhancer;
      (b) a rearranged antibody variable region encoding a rearranged antibody V domain (eg, a human V domain);
      (c) an exogenous antibody light chain constant region (eg, a human Cκ or Cλ) encoding a light chain C domain; and
      (d) an endogenous antibody light chain constant region of the cell (eg, wherein the cell is a rodent, mouse or rat cell)
      wherein the locus is operable to express an antibody chain comprising (in N- to C-terminal direction) said rearranged V domain and said C domain.
   For example, the locus comprises (in 5' to 3' direction)
      (a) an endogenous light chain intronic enhancer of the cell (eg, wherein the cell is a rodent, mouse or rat cell);
      (b) a rearranged antibody variable region encoding a rearranged antibody V domain (eg, a human V domain);
      (c) an antibody light chain constant region encoding a light chain C domain (eg, a human C domain, such as when the cell is a rodent, mouse or rat cell); and
      (d) optionally an endogenous antibody light chain constant region of the cell;
      wherein the locus is operable to express an antibody chain comprising (in N- to C-terminal direction) said rearranged V domain and said C domain.
   Preferably, the intronic enhancer is an endogenous enhancer of the cell (eg, wherein the cell is a rodent, mouse or rat cell), preferably a kappa locus Eiκ enhancer. Preferably, the cell is a cell of a first species or strain (eg, a mouse or rat species or strain) and the intronic enhancer of the invention comprises a wild-type intronic enhancer sequence of said species or strain. For example, the cell is a F1H4, 129 or C57BL6 strain cell and the enhancer is an intronic enhancer of said strain; optionally wherein the enhancer is a Eiκ and/or the variable region is an insert upstream of an endogenous kappa constant region of the cell (eg, between an endogenous variable region of the cell (such as a human or mouse variable region in a mouse cell) and an endogenous Cκ (eg, mouse Cκ) of a kappa locus of the cell).
2. The cell of Statement 1, wherein the locus is a kappa light chain locus and the enhancer is Eiκ.
   In an example the enhancer is Eiκ, eg, and the locus is a lambda light chain locus. Preferably in this example or Statement, the V domain and C domain are a human Vκ and a human Cκ, eg, as comprised by a known antibody that binds a predetermined antigen (eg, the first antigen mentioned above); or the V domain and C domain are a human Vλ and a human Cλ, eg, as comprised by a known antibody that binds a predetermined antigen (eg, the first antigen mentioned above), wherein example, the cell is a rodent, rat our mouse cell.
   For example, the locus is a kappa light chain locus and the enhancer is Eiκ, the cell is a mouse cell, and the V domain and C domain are a human Vκ and a human Cκ, eg, as comprised by a known antibody that binds a predetermined antigen (eg, the first antigen mentioned above). For example, the locus is a kappa light chain locus and the enhancer is Eiκ, the cell is a mouse cell, and the V domain and C domain are a human Vλ and a human Cλ, eg, as comprised by a known antibody that binds a predetermined antigen (eg, the first antigen mentioned above).
   In an embodiment, the cell is a rodent, mouse or rat cell and the enhancer is a rodent, mouse or rat intronic enhancer (eg, Eiκ) respectively.
   In an example the light chain constant region is a Cκ and the locus comprises a kappa 3' enhancer that is operably connected 3' of the Cκ. In another example, the light chain constant region is a Cλ.
   Optionally, the constant region is a human constant region. Optionally, the constant region is a mouse constant region. Optionally, the constant region is a rat constant region.
   Optionally, the constant region is a human Cκ. Optionally, the constant region is a mouse Cκ. Optionally, the constant region is a rat Cκ.
   Optionally, the constant region is a human Cλ. Optionally, the constant region is a mouse Cλ. Optionally, the constant region is a rat Cλ.
   Optionally, the enhancer is a human enhancer. Optionally, the enhancer is a mouse enhancer. Optionally, the enhancer is a rat enhancer.
   Optionally, the enhancer is a human Eiκ enhancer. Optionally, the enhancer is a mouse Eiκ enhancer. Optionally, the enhancer is a rat Eiκ enhancer.
   Optionally, the enhancer is a human lambda enhancer. Optionally, the enhancer is a mouse lambda enhancer. Optionally, the enhancer is a rat lambda enhancer.
   In an example, the enhancer is immediately flanked 5' and/or 3' by human kappa or lambda variable region intron sequence (eg, wherein the enhancer is a mouse enhancer and the cell is a mouse or rat cell; or wherein the enhancer is a rat enhancer and the cell is a mouse or rat cell). Optionally, each flanking sequence is a sequence comprised by a human VL intron between a signal peptide-encoding sequence and a VL-encoding sequence of a nucleotide sequence encoding said V domain (wherein the V domain is said VL). For example, the locus comprises a genomic fragment of a B-cell that is capable of expressing the VL domain (or a light chain comprising the VL), wherein the fragment comprises (in 5' to 3' order) a promoter, a VL signal peptide-encoding sequence, a first intron sequence, said enhancer, as second intron sequence, the variable region and optionally the constant region, wherein the first and second intron sequences are naturally contiguous in the B-cel genome or are spaced by no more than 500, 400, 300, 200, 100, 50 or 20bp (bp=base pairs).
   For example, the locus comprises a nucleotide sequence that encodes a rearranged light chain (eg, a kappa or lambda chain) of an antibody (eg, wherein the antibody specifically binds to a predetermined antigen or epitope). For example, said nucleotide sequence is comprised by a cloned genomic fragment from an antibody-producing cell, such as a B-cell or hybridoma, that expresses the antibody. In an example, the nucleotide sequence is a cloned genomic sequence of said antibody-producing cell comprising a sequence from a promoter of said variable region to (and including) the variable region and optionally also to (and including) the constant region operably linked to the variable region. For example, the cell is a mouse or rat cell and the genomic fragment comprises the sequence of a genomic fragment of a kappa antibody-producing cell that is specific for a predetermined antigen, wherein the genomic sequence comprises the nucleotide sequence from a promoter of said variable region to (and including) the variable region; optionally wherein the sequence is comprised by the locus of the invention 5' of a human Cκ constant region of the locus. For example, the cell is a mouse or rat cell and the genomic fragment comprises the sequence of a genomic fragment of a lambda antibody-producing cell that is specific for a predetermined antigen, wherein the genomic sequence comprises the nucleotide sequence from a promoter of said variable region to (and including) the variable region; optionally wherein the sequence is comprised by the locus of the invention 5' of a human Cλ or Cκ constant region of the locus. In an example, the nucleotide sequence is an insert in a kappa or lambda locus of the cell, eg, operably linked 5' of an endogenous 3' enhancer of the kappa or lambda locus of the cell.
   In an example of any cell, vertebrate or other aspect of the invention herein, endogenous lambda and/or kappa chain expression of the cell or vertebrate is inactive or substantially inactive. In an embodiment, inactivation is more than 50% (ie, 50% or less of the antibodies or transcripts are of a said endogenous antibody chain), 60%, 70%, 80%, 85%, 90%, 95%, 96%, 97%, 98% or 99%. For example, in an embodiment, said endogenous lambda chain expression is substantially inactive such that no more than 85%, 90%, 95%, 96%, 97%, 98% or 99% of the lambda chain repertoire of the vertebrate is provided by endogenous lambda chains. For example, endogenous lambda chain expression is inactive such that none or substantially none of the lambda chain repertoire of the vertebrate is provided by endogenous lambda chains. For example, in an embodiment, said endogenous kappa chain expression is substantially inactive such that no more than 85%, 90%, 95%, 96%, 97%, 98% or 99% of the kappa chain repertoire of the vertebrate is provided by endogenous kappa chains. For example, endogenous kappa chain expression is inactive such that none or substantially none of the kappa chain repertoire of the vertebrate is provided by endogenous kappa chains.
   In an example, the locus of the invention is a modification of an endogenous kappa locus allele of the cell, and optionally the cell is heterozygous or homozygous for said modification. In an embodiment, the cell is heterozygous for the modification and the cell comprises a wild-type or humanised locus at the other kappa locus allele.
   In an example, the locus of the invention is a modification of an endogenous lambda locus allele of the cell, and optionally the cell is heterozygous or homozygous for said modification. In an embodiment, the cell is heterozygous for the modification and the cell comprises a wild-type or humanised locus at the other lambda locus allele.
3. The cell of Statement 1, wherein the cell is a mouse cell, locus is a lambda light chain locus and the enhancer is a mouse lambda 2-4 or 4-10 enhancer.
4. The cell of any preceding Statement, wherein
   (a) the rearranged V domain is a Vκ and the C domain is a Cκ;
   (b) the rearranged V domain is a Vκ and the C domain is a Cλ;
   (c) the rearranged V domain is a Vλ and the C domain is a Cλ; or
   (d) the rearranged V domain is a Vλ and the C domain is a Cκ.
5. A cell comprising an antibody heavy chain locus (first locus), wherein the locus comprises (in 5' to 3' direction)
   (a) a heavy chain intronic enhancer (Eµ);
   (b) a rearranged antibody variable region encoding a rearranged antibody V domain (eg, a VL, Vλ or Vκ, eg, a VH);
   (c) an optional switch sequence; and
   (d) an antibody heavy chain constant region encoding a heavy chain CH1 domain;
   wherein the locus is operable to express an antibody chain comprising (in N- to C-terminal direction) said rearranged V domain and said CH1 domain.
   Optionally, the constant region encodes an antibody Fc (eg, in N- to C-terminal direction: CH1 - optional hinge - CH2-CH3).
   Optionally, the CH1 is a human or mouse CH1, eg, when the cell is a mouse cell.
   In an example, the constant region encodes an antibody Fc (eg, in N- to C-terminal direction: CH1 - optional hinge - CH2-CH3) there is no further C domain-encoding nucleotide sequence 3' of said constant region.
6. The cell of any preceding Statement, wherein the locus comprises (in 5' to 3' direction)
   (a) a promoter operable for promoting transcription of the variable region;
   (b) a nucleotide sequence encoding a variable domain signal peptide;
   (c) said intronic enhancer;
   (d) said rearranged antibody variable region; and
   (e) said antibody light chain constant region,
   Wherein the locus is operable to express RNA transcripts encoding (in N- to C-terminal direction) said signal peptide sequence fused to the amino acid sequence of said variable domain.
   Optionally, the promoter and/or the signal peptide-encoding sequence are those that are operable with the variable region (or a V gene segment sequence thereof) in a wild-type cell (eg, a human cell when the V region is a human V region comprised by the human cell), B-cell or hybridoma, such as a B-cell or hybridoma that comprises a sequence identical to said variable region for expressing antigen-specific V domains.
7. The cell of any preceding Statement, wherein the cell is an ES (eg, a 129, C57BL/6, AB2.1, AB2.2, JM8 or F1H4 type ES cell), iPS, hybridoma or B-cell.
8. The cell of any preceding Statement, wherein the rearranged V domain is a kappa or lambda V domain, eg, a rearranged human kappa or lambda V domain.
9. The cell of any preceding Statement, wherein the variable region encodes for a V domain that has a binding specificity for a first predetermined antigen or a first epitope (eg, when paired with another rearranged V domain), wherein the locus is operable to express an antibody chain that comprises a V domain that retains said specificity (eg, when paired with said other V domain).
   For example, the variable region encodes for a VL domain that has a binding specificity for a first predetermined antigen or a first epitope (eg, when paired with a rearranged VH domain), wherein the locus is operable to express an antibody light chain that comprises a VL domain that retains said specificity (eg, when paired with said VH domain). For example, the VL and VH are identical to the VL and VH of a known antibody that is capable of specifically binding to a predetermined antigen.
10. The cell of any preceding Statement, wherein the cell comprises a second antibody locus that is operable to express a second antibody chain, wherein the second chain comprises a second rearranged V domain that forms a binding site with the first rearranged V domain, wherein the binding site is capable of specifically binding to a predetermined antigen or a epitope.
   For example, the first locus encodes a VL domain and the second locus encodes a VH domain, wherein the VL and VH are capable of forming a VH/VL binding site for said predetermined antigen or epitope.
11. The cell of Statement 10 when dependent from Statement 9, wherein the predetermined antigens are different and said other and second V domains are different; or wherein the epitopes are different and said other and second V domains are different.
   For example, the eptiopes are different epitopes comprised by the same antigen.
12. The cell of Statement 11, wherein
   (a) the first domain is a VL (eg, Vκ or Vλ) domain and the second and said other V domains are VH domains; or
   (b) wherein the first domain is a VH domain and the second and said other V domains are VL (eg, both Vκ or Vλ) domains.
13. The cell of any preceding Statement, wherein the variable region is within 0.5, 0.4, 0.3, 0.2 or 0.1 kb 3' of the enhancer.
   Optionally, the variable region is spaced from the intronic enhancer by no more than 0.5, 0.4, 0.3, 0.2 or 0.1 kb.
14. The cell of any preceding Statement, wherein the locus comprises a second enhancer that is 3' of the constant region.
   Optionally, the locus (eg, according to Statement 5) is a heavy chain locus, and the second enhancer is a heavy chain 3' enhancer.
   Optionally, the locus (eg, according to Statement 1) is a kappa chain locus, and the second enhancer is a kappa chain 3' enhancer.
   Optionally, the locus (eg, according to Statement 1) is a lambda chain locus, and the second enhancer is a lambda chain 3' enhancer, eg, a mouse lambda 3-1 enhancer.
15. The cell of any preceding Statement, wherein the cell is a non-human mammal or rodent (eg, a mouse or rat) cell.
16. The cell of any preceding Statement, wherein said constant region is at an endogenous antibody locus of the cell.
   Optionally, a further variable region (eg, a mouse variable region or a human variable region) is 5' of the intronic enhancer; for example, the further variable region is inactive for expression of variable domains. Optionally, no variable region (eg, a mouse variable region or a human variable region) is 5' of the intronic enhancer. Optionally, the further variable region comprises a lesion (eg, a J region lesion or is devoid of J gene segments) or a neo (neomycin resistance) marker, thereby inactivating rearrangement in said further variable region.
17. The cell of Statement 16 when dependent from Statement 1, wherein said endogenous locus is an endogenous kappa chain locus.
18. The cell of Statement 16 when dependent from Statement 5, wherein said endogenous locus is an endogenous heavy chain locus.
19. The cell of any one of Statements 1 to 15, wherein the locus is comprised by a transgene that is comprised by the genome of the cell at a position outside an endogenous antibody locus, (eg, at Rosa 26).
   Optionally, the cell genome comprises a plurality of said transgenes.
20. The cell of any preceding Statement, wherein the cell is homozygous for said locus.
   Alternatively, the cell is heterozygous for said locus.
21. The cell of any preceding Statement when dependent from Statement 1, wherein the cell genome comprises a second antibody locus, wherein the second locus is an unrearranged antibody heavy chain locus comprising (in 5' to 3' direction)
   (a) one or more VH gene segments;
   (b) one or more DH gene segments;
   (c) one or more JH gene segments; and
   (d) a heavy chain constant region encoding one or more CH domains;
   Wherein the heavy chain locus is operable to express a plurality of heavy chains, optionally comprising a plurality of antigen specificities or affinities, each said heavy chain being capable of pairing with an antibody chain (eg, a kappa or lambda light chain) encoded by the first locus to produce paired chains that comprise an antigen binding site.

Preferably, the VH (eg, the VH, DH and JH) gene segments are human gene segments. Optionally, the CH is a rodent, eg, a mouse or rat, or a human CH (eg, when the cell is a mouse or rat cell).

Optionally, in an alternative the cell of the invention comprises a heavy chain transgene comprising an unrearranged heavy chain variable region (eg, a human V region) operably connected 5' of a heavy chain constant region (eg, a mouse, rat or human constant region) for expressing a plurality of different heavy chains.

In an example, the second locus according to Statement 21 or said heavy chain variable region of the transgene comprises at least 5, 10, 15 or all human VH gene segments of the group consisting of IGHV1-3, IGHV1-8, IGHV1-18, IGHV1-46, IGHV3-7, IGHV3-9, IGHV3-11, IGHV3-15, IGHV3-20, IGHV3-21, IGHV3-23, IGHV3-30, IGHV3-33, IGHV3-48, IGHV3-53, IGHV4-4, IGHV4-31, IGHV4-34, IGHV4-39, IGHV4-59, IGHV4-61, IGHV5-51, IGHV6-1 and IGHV7-4-1.

In an example, the first locus of the invention encodes a light chain, wherein the light chain pairs with or is capable of pairing with at least 5, 10, 15 or all human VH gene segments of the group consisting of IGHV1-3, IGHV1-8, IGHV1-18, IGHV1-46, IGHV3-7, IGHV3-9, IGHV3-11, IGHV3-15, IGHV3-20, IGHV3-21, IGHV3-23, IGHV3-30, IGHV3-33, IGHV3-48, IGHV3-53, IGHV4-4, IGHV4-31, IGHV4-34, IGHV4-39, IGHV4-59, IGHV4-61, IGHV5-51, IGHV6-1 and IGHV7-4-1.

In an example, the light chain pairs with or is capable of pairing with human VH gene segments IGHV1-3, IGHV1-8, IGHV1-18, IGHV1-46, IGHV3-7, IGHV3-9, IGHV3-11, IGHV3-15, IGHV3-20, IGHV3-21, IGHV3-23, IGHV3-30, IGHV3-33, IGHV3-48, IGHV3-53, IGHV4-4, IGHV4-31, IGHV4-34, IGHV4-39, IGHV4-59, IGHV4-61, IGHV5-51, IGHV6-1 and IGHV7-4-1 in a vertebrate of the invention.

In an example, the second locus according to Statement 21 or said heavy chain variable region of the transgene comprises at least 5, 10, 15 or all human VH gene segments of the group consisting of IGHV1-3*01, IGHV1-8*01, IGHV1-18*01, IGHV1-46*03, IGHV3-7*01, IGHV3-9*01, IGHV3-11*01, IGHV3-15*01, IGHV3-20*d01, IGHV3-21*03, IGHV3-23*04, IGHV3-30*18, IGHV3-33*01, IGHV3-48*02, IGHV3-53*01, IGHV4-4*02, IGHV4-31*03, IGHV4-34*01, IGHV4-39*01, IGHV4-59*01, IGHV4-61*01, IGHV5-51*01, IGHV6-1*01 and IGHV7-4-1*01.

In an example, the first locus of the invention encodes a light chain, wherein the light chain pairs with or is capapble of pairing with at least 5, 10, 15 or all human VH gene segments of the group consisting of IGHV1-3*01, IGHV1-8*01, IGHV1-18*01, IGHV1-46*03, IGHV3-7*01, IGHV3-9*01, IGHV3-11*01, IGHV3-15*01, IGHV3-20*d01, IGHV3-21*03, IGHV3-23*04, IGHV3-30*18, IGHV3-33*01, IGHV3-48*02, IGHV3-53*01, IGHV4-4*02, IGHV4-31*03, IGHV4-34*01, IGHV4-39*01, IGHV4-59*01, IGHV4-61*01, IGHV5-51*01, IGHV6-1*01 and IGHV7-4-1*01.

In an example, the light chain pairs with or is capable of pairing with human VH gene segments IGHV1-3*01, IGHV1-8*01, IGHV1-18*01, IGHV1-46*03, IGHV3-7*01, IGHV3-9*01, IGHV3-11*01, IGHV3-15*01, IGHV3-20*d01, IGHV3-21*03, IGHV3-23*04, IGHV3-30*18, IGHV3-33*01, IGHV3-48*02, IGHV3-53*01, IGHV4-4*02, IGHV4-31*03, IGHV4-34*01, IGHV4-39*01, IGHV4-59*01, IGHV4-61*01, IGHV5-51*01, IGHV6-1*01 and IGHV7-4-1*01 in a vertebrate of the invention.

Optionally, the cell is a mouse or rat ES cell that is capable of developing into a mouse or rat respectively, wherein the mouse or rat is capable of expressing a plurality of heavy chains, optionally comprising a plurality of antigen specificities or affinities, each said heavy chain being capable of pairing with an antibody chain (eg, a kappa or lambda light chain) encoded by the first locus to produce paired chains that comprise an antigen binding site.

22. The cell of any one of Statements 1 to 20 when dependent from Statement 5, wherein the cell comprises one or more further antibody loci, each further locus being capable of expressing a light chain, each said light chain being capable of pairing with an antibody chain (eg, a VH-Fc) encoded by the first locus to produce paired chains that comprise an antigen binding site.

Optionally, the cell is a mouse or rat (or other non-human vertebrate) ES cell that is capable of developing into a mouse or rat (or other vertebrate) respectively, wherein the mouse or rat (or vertebrate) is capable of expressing one or more light chains, optionally comprising a plurality of antigen specificities or affinities, each said light chain being capable of pairing with an antibody chain (eg, a VH-Fc) encoded by the first locus to produce paired chains that comprise an antigen binding site. This may be useful in the practice of the following method of producing a bi-specific antibody:-
A method comprising
(i) Providing a non-human vertebrate (eg, a mouse or rat) according to the immediately preceding paragraph, wherein the variable region of the first locus encodes a VH domain of an antibody that specifically binds to a first predetermined antigen;
(ii) immunising the vertebrate with a second predetermined antigen, wherein the vertebrate expresses a plurality of said light chains, wherein VL domains of the chains pair with copies of said VH to form VH/VL antigen binding sites which specifically bind to the second antigen;
(iii) Isolating one or more VH/VL pairs that bind to the first and second antigens; or obtaining one or more B-cells or hybridomas that express said VH/VL pairs that bind the antigens; or obtaining a nucleotide sequence encoding a VL of a said pair and/or a nucleotide sequence of a VH of a said pair;
(iv) Optionally, inserting the nucleotide sequences into one or more expression vectors or one or more host cells for expressing bispecific antibodies comprising VH/VL paired binding sites, where each binding site is capable of specifically binding to the first and second antigens; and
(v) Optionally expressing and isolating said bispecific antibodies.

In an example, the vertebrate of step (i) is a chimaera as described herein, wherein the chimaera expresses said one or more light chains.

There is also provided:-
A method of producing a bispecific antibody, comprising expressing from a host cell line a plurality of antibodies, wherein the host cell line is obtained by steps (i) to (iv) or is cultured from cells so obtained.

There is also provided:-
A method of producing a bispecific antibody, comprising expressing from a host cell line a plurality of antibodies, wherein each host cell genome comprises an expressible nucleotide sequence encoding a heavy chain variable domain of the bispecific antibody, wherein the variable domain is capable of specifically binding a first antigen, the nucleotide sequence being identical (or at least 80, 85, 90 or 95% identical) to a nucleotide sequence of a B-cell of a vertebrate of the invention wherein the vertebrate has been immunised with the first antigen. In an example, each host cell genome comprises a a nucleotide sequence encoding light chain variable domain that is identical (or at least 80, 85, 90 or 95% identical) to a nucleotide sequence of the B-cell, wherein the heavy and light chains domains form a paired VH/VL first antigen binding site. Optionally, the method comprises isolating a plurality of such bispecific antibodies, and optionally further formulating the antibodies with a carrier, diluent or excipient to produce a composition (such as a pharmacetical composition). The invention also relates to such a plurality of antibodies or composition obtained or obtainable by this method or any other method of the invention.

In an alternative, instead of the first locus encoding a VH, the first locus instead encodes a VL or light chain and the vertebrate is capable of expressing one or more heavy chains, optionally comprising a plurality of antigen specificities or affinities, each said heavy chain being capable of pairing with an antibody chain (eg, a light) encoded by the first locus to produce paired chains that comprise an antigen binding site. Such a vertebrate may be used *mutatis mutandis* in a method comprising steps (i) *etc,* wherein the VL is a VL of an antibody that specifially binds to the first antigen.

In this way, chain shuffling can be performed *in vivo* according to the invention, wherein the starting point can be a fixed, predetermined VH or VL of an antibody of known antigen binding specificity. The end result is an antibody, wherein VH/VL binding sites are themselves bispecific for first and second antigens (or alternatively first and second epitopes of the same antigen).

23. The cell of any preceding Statement, wherein each variable region or gene segment is human, and optionally said constant region is human.

Optionally, each gene segment is a human germline gene segment.

24. The cell of any preceding Statement, wherein the cell is a mouse cell and each constant region is a mouse, rat or human constant region.

For example, each constant region is a human constant region.

25. The cell of any preceding Statement, wherein each said enhancer is an endogenous enhancer of the cell.

In an example, the intronic enhancer is of the same species or strain as the cell, comprises the endogenous enhancer sequence of the cell, or comprises an insert into the cell genome (eg, an insert that comprises an enhancer sequence that is identical to the endogenous enhancer sequence).

26. The cell of any preceding Statement, wherein the cell is a mouse cell and each said enhancer is a mouse enhancer.

For example, the enhancer is a 129 hybrid or C57BL6 hybrid strain enhancer (and optionaly the cell is a 129 hybrid or C57BL6 hybrid strain respectively). For example, the enhancer is a 129 enhancer and the cell is a F1H4 or AB2.1 cell.

27. The cell of any preceding Statement, wherein
(a) the cell is a mouse cell, the variable region is a human variable region, the intronic enhancer is a mouse intronic enhancer (eg, sequence identical to an endogenous mouse intronic enhancer) at an endogenous antibody locus (eg, a kappa locus) of the cell and said constant region is a mouse, rat or human constant region (eg, an endogenous mouse constant region of the cell); or
(b) the cell is a rat cell, the variable region is a human variable region, the intronic enhancer is a rat intronic enhancer (eg, sequence identical to an endogenous rat intronic enhancer) at an endogenous antibody locus (eg, a kappa locus) of the cell and said constant region is a mouse, rat or human constant region (eg, an endogenous rat constant region of the cell).

For example, the cell is a mouse cell, the variable region is a human variable region, the intronic enhancer comprises a sequence identical to an endogenous mouse intronic enhancer and is at an endogenous kappa locus of the cell and said constant region is a human constant region.

For example, the cell is a mouse F1H4 cell, the variable region is a human variable region, the intronic enhancer comprises a sequence identical to a mouse 129 Eiκ intronic enhancer and is at an endogenous kappa locus of the cell and said constant region is a human constant region. For example, the cell is a mouse AB2.1 cell, the variable region is a human variable region, the intronic enhancer comprises a sequence identical to a mouse 129 Eiκ intronic enhancer and is at an endogenous kappa locus of the cell and said constant region is a human constant region.

For example, the cell is a mouse F1H4 cell, the variable region is a human lambda variable region, the intronic enhancer comprises a sequence identical to a mouse 129 Eiκ intronic enhancer and is at an endogenous kappa locus of the cell and said constant region is a human lambda constant region. For example, the cell is a mouse AB2.1 cell, the variable region is a human lambda variable region, the intronic enhancer comprises a sequence identical to a mouse 129 Eiκ intronic enhancer and is at an endogenous kappa locus of the cell and said constant region is a human lambda constant region.

For example, the cell is a mouse F1H4 cell, the variable region is a human kappa variable region, the intronic enhancer comprises a sequence identical to a mouse 129 Eiκ intronic enhancer and is at an endogenous kappa locus of the cell and said constant region is a human kappa constant region. For example, the cell is a mouse AB2.1 cell, the variable region is a human kappa variable region, the intronic enhancer comprises a sequence identical to a mouse 129 Eiκ intronic enhancer and is at an endogenous kappa locus of the cell and said constant region is a human kappa constant region

28. The cell of any preceding Statement, wherein the rearranged variable region is a rearrangement of
(a) human IGLV3-21 and IGLJ3 (eg, IGLV3-21*01 and IGLJ3*02) and optionally operably connected to human germline IGLV3-21 (eg, IGLV3-21*01) promoter and/or signal peptide-encoding nucleotide sequence;
(b) human IGK1-39 and IGKJ1 or 5 and optionally operably connected to human germline IGKV1-39 promoter and/or signal peptide-encoding nucleotide sequence;
(c) human IGK3-20 and IGKJ1 or 5 and optionally operably connected to human germline IGKV3-20 promoter and/or signal peptide-encoding nucleotide sequence;
(d) a human VpreB and Jλ5 and optionally operably connected to human germline VpreB promoter and/or signal peptide-encoding nucleotide sequence.

29. A transgenic non-human vertebrate comprising a plurality of cells according to any preceding Statement.

For example, the vertebrate is an embryo comprising at least 20, 30, 40 or 50% of its cells as cells of the invention. For example, the vertebrate is rodent (eg, mouse or rat) child comprising at least 20, 30, 40 or 50% of its cells as cells of the invention, eg, as indicated by percentage of coat colour (eg, total area of albino versus aguti coat colour).

30. The vertebrate of Statement 29, wherein the germline of said vertebrate comprises a first locus (and optionally the second locus) as defined in any one of Statements 1 to 28.

For example, the invention provides a transgenic non-human vertebrate chimaera comprising a plurality of cells according to any preceding Statement. Optionally, the germline of said chimaera (or the chimaera of Statement 31) does not comprise a first locus (and optionally also not the second locus) as defined in any one of Statements 1 to 28. Thus, with the present invention the time and effort required to genereate vertebrates that show germline transmission of the locus is avoided, as chimaeras can be simply generated and used without germline transmission, which is a benefit over prior art configurations.

31. The vertebrate of Statement 29, wherein the vertebrate is a chimaera of said cells and a plurality of other cells that do not comprise the first locus (eg, wherein the other cells comprise wild-type antibody loci), wherein the germline of the vertebrate does not comprise a first locus.

Optionally, said other cells comprise a RAG knock-out. This is useful as productive mature B-cells in the chimaera will result only from cells of the invention, thereby removing unwanted background. Here, productive refers to B-cells that can express antibodies that contribute to the antibody repertoire of the vertebrate.

In an example, the cells of the invention are F1H4, AB2.1, AB2.2, 129 or C57BL6 strain cells and said other cells are F1H4, AB2.1, AB2.2, 129 or C57BL6 strain cells (eg, progeny cells of a wild-type blastocyst or pre-morula embryo; or a RAG knock-out blastocyst or pre-morula embryo).

32. The vertebrate of any one of Statements 29 to 31, comprising a first plurality of cells and a second plurality of cells, wherein the cells of the first plurality comprise the same first identical antibody locus, and the cells of the second plurality comprise the same additional identical antibody locus, wherein each said antibody locus is according to the first locus of any one of Statements 1 to 28, wherein the vertebrate is capable of expressing a first plurality of antibody chains from said first identical locus and a second plurality of antibody chains from said additional locus, and wherein the antibody locus of the first cells is different from the antibody locus of the second cells.

33. The vertebrate of Statement 32, wherein the vertebrate comprises a third plurality of cells, wherein the cells of the third plurality comprise the same further antibody locus, wherein the further locus is according to any one of Statements 1 to 28 and is different from said antibody loci of the first and second cells, wherein the vertebrate is capable of expressing a third plurality of antibody chains from the further locus.

34. The vertebrate of Statement 32 or 33, wherein the vertebrate is a chimaera of said cells and a plurality of other cells that do not comprise a first locus according to any one of Statements 1 to 28 or said additional or further loci (eg, wherein the other cells comprise wild-type antibody loci).

35. A transgenic non-human vertebrate whose antibody light chain repertoire is at least 95, 96, 97, 98 or 99% pure (or is 100% pure) for a single rearranged VL domain species.

This, therefore, means that all VL expressed by the vertebrate are said VL domain species, or only less than 5% of VL are of another species.

In an example, a vertebrate herein is naive. In another example, the vertebrate is antigen immunised (eg, human antigen immunised).

Purity may, for example, be determined from a blood sample or VL-encoding nucleotide sequence sample of the vertebrate, using standard NGS and optionally with PCR amplification of VL-encoding nucleotide sequences comprised by the sample. Optionally, B-cells or hybridomas from or produced from the sample are sorted into individual wells of one or more plates and then subjected to optional PCR of the VL-encoding nucleotide sequence thereof, followed by NGS to determine the sequence of the nucleotide sequence in each well. The sequences are analysed to determine the proportion that encode said single rearranged VL domain species.

In an example, the VL species is a human Vκ. In an example, the VL species is a human Vλ.

36. The vertebrate of Statement 35, wherein the repertoire is at least 99% pure for a single rearranged VL domain species.

37. The vertebrate of Statement 35 or 36, wherein the remainder of the light chains of the light chain repertoire comprise mutants of said VL domain species.

38. A transgenic non-human vertebrate whose antibody light chain repertoire comprises VL domains derived from the recombination of a first VL gene segment and a first JL gene segment, wherein at least 95% of all VL domains derived from said recombination comprise the same VL amino acid sequence.

In another embodiment, the invention provides a transgenic non-human vertebrate wherein at least 95, 96, 97 98 or 99% (or 100%) of all VL domains of the antibody light chain repertoire of the vertebrate are encoded by the same light chain variable region sequence comprised by the genome of the vertebrate.

In another embodiment, the invention provides a transgenic non-human vertebrate wherein at least 95, 96, 97 98 or 99% (or 100%) of all VL domains of the human VL repertoire of the vertebrate are encoded by the same light chain variable region sequence comprised by the genome of the vertebrate.

39. The vertebrate of Statement 38, wherein at least 99% of all VL domains derived from said recombination comprise the same VL amino acid sequence.

40. A plurality of spleen, bone marrow, B-cells or blood cells of a vertebrate according to any one of Statements 29 to 39 or said other embodiments of Statement 38 comprising a plurality of first loci according to any one of Statements 1 to 28.

41. A population of spleen, bone marrow, B-cells, hybridomas, CHO cells, HEK cells, MEF cells, COS cells, HeLa cells or blood cells, wherein each cell is according to Statement 1 or any one of Statements 2 to 28 when dependent from Statement 1; and optionally, wherein the cells express at least 10 different antibody species wherein
(a) at least 95, 96, 97, 98 or 99% (or 100%) of the antibodies share the same light chain VL domain and the population comprises at least 10 different VH domain species; or
(b) the antibodies comprise VL domains derived from the recombination of a first VL gene segment and a first JL gene segment (eg, human germline Vκ and Jκ gene segments or human germline Vλ and Jλ gene segments), wherein at least 95% of all said VL domains derived from said recombination comprise the same VL amino acid sequence and the population comprises at least 10 different VH domain species.

Optionally, the cells are HEK293 cells, eg, HEK293T or S cells, COS-1 or COS-7 cells.

Optionally, the cells express at least 100, 1000, 10000, 100000, 1000000, 10000000, 100000000, or 1000000000 different antibody species. Optionally, the population comprises at least 100, 1000, 10000, 100000, 1000000, 10000000, 100000000, or 1000000000 different VH domain species.

42. A polyclonal antibody population, wherein at least 95, 96, 97, 98 or 99% (or 100%) of the antibodies comprised by the population share the same light chain VL domain and the population comprises at least 10 different VH domain species.

Optionally, the population comprises at least 100, 1000, 10000, 100000, 1000000, 10000000, 100000000, or 1000000000 different VH domain species.

In an example, an antibody population herein is contained in a sterile or medical container, in a test tube, petri dish or a flask. Optionally the population is mixed with B-cells, wherein the B-cells comprise a locus according to the invention.

43. A polyclonal antibody population, comprising VL domains derived from the recombination of a first VL gene segment and a first JL gene segment (eg, human germline Vκ and Jκ gene segments or human germline Vλ and Jλ gene segments), wherein at least 95, 96, 97, 98 or 99% (or 100%) of all said VL domains derived from said recombination comprise the same VL amino acid sequence and the population comprises at least 10 different VH domain species. Optionally, the population comprises at least 100, 1000, 10000, 100000, 1000000, 10000000, 100000000, or 1000000000 different VH domain species.

44. A method of identifying or obtaining an antibody, an antibody variable domain, a nucleotide sequence (eg, DNA or RNA sequence) encoding an antibody or an antibody variable domain, or an expression vector or host cell that is capable of expressing the antibody or domain, wherein the antibody or domain is capable of specifically binding to a target antigen, the method comprising
(a) contacting the cell population of Statement 41, or the antibody population of 42 or 43 with said antigen (eg, immobilised on a solid support);
(b) binding antibodies expressed or comprised by said population to said antigen; and
(c) isolating or identifying one or more antibodies that bind to the antigen, or isolating or identifying a VH and/or a VL domain of a said one or more antibody; or identifying a nucleotide sequence encoding a said VH or VL; and
(d) optionally
   (i) correlating a said identified antibody or domain with a nucleotide sequence (eg, DNA, cDNA, RNA or mRNA sequence) encoding therefor, thereby identifying said sequence;
   (ii) amplifying said sequence (eg, using PCR) and inserting the sequence into an expression vector or a host cell genome for expression of the encoded antibody or domain; and
   (iii) optionally expressing and isolating said antibody or domain (eg, wherein the domain is comprised by an antibody chain),
   wherein steps (i) and (ii) can be carried out in any order.

NGS may be used, for example, in said correlating.

45. The method of Statement 44, comprising using the method to amplify the VH and VL nucleotide sequences encoding the VH and VL domains of the identified antibody and further comprising inserting the VH and VL nucleotide sequences into the vector or cell for co-expression of the VH and VL to produce paired VH/VL binding sites that are capable of binding to the antigen, and expressing (and optionally isolating) the binding sites (eg, comprised by antibodies encoded by the vector or cell).

46. The method of Statement 45, wherein the method comprises expressing the VH and VL in the presence of a further species of VH domain, wherein the VL and the further VH form further paired VH/VL sites that are capable of binding to a further antigen, wherein the further antigen and the antigen recited in Statement 45 are different, the method comprising co-expressing (and optionally isolating) the binding sites (eg, comprised by bi-specific antibodies encoded by the vector or cell, wherein the bi-specific antibodies comprise a respective binding site for each said antigen).

47. The method of Statement 46, wherein the bi-specific antibodies are 4-chain antibodies comprising a first heavy chain-light chain pair and a second heavy chain-light chain pair, wherein
(a) the light chain in the first and second pairs comprises said VL domain,
(b) the heavy chain of the first pair comprises the VH domain recited in Statement 45, wherein the VH domain forms a VH/VL binding site with said VL domain and wherein the binding site is capable of specifically binding to said antigen recited in Statement 45;
(c) the heavy chain of the second pair comprises said further VH domain that forms a VH/VL binding site with said VL domain and wherein the binding site is capable of specifically binding to said further antigen recited in Statement 46.

48. The method of Statement 47, wherein each 4-chain antibody comprises mutated heavy chain constant regions to promote pairing of the heavy chains of the first and second heavy-light pairs; optionally wherein the mutations are knob-in-hole mutations or charge pair mutations.

For example, each constant region is encoded by a heavy chain constant region sequence comprised by an expression vector.

Advantageously, in an example the heavy chains of the first pair have a first pl and the heavy chains of the second pair have a second pl, wherein the pl values are different, for example differ by 0.5-1.5 pl units, eg, differ by 0.5-1 pl units. This is useful for separating the first chains from the second chains during in vitro purification of chains, as is useful during antibody manufacture and scale-up. For example, each pl is in the range of 5 to 6.5.

49. A plurality of B-cells, hybridomas, CHO cells, HEK cells, MEF cells, COS cells, HeLa cells that expresses the polyclonal antibody population of Statement 42 or 43.

Optionally, the cells are HEK293 cells, eg, HEK293T or S cells, COS-1 or COS-7 cells.

50. The method of any one of Statements 44 to 48, wherein the method comprises obtaining VL and/or VH-encoding nucleotide sequences from the cells of Statement 41 or 47, and in step (d) (i) using the nucleotide sequences to correlate one or more thereof with one or more VH and/or VL domains of said antibodies that bind the antigen.

51. A method obtaining a nucleotide sequence encoding an antibody or an antibody variable domain, wherein the antibody or domain is capable of specifically binding to a target antigen, the method comprising
(a) obtaining VL and/or VH-encoding nucleotide sequences from the cells of Statement 41 or 47;
(b) amplifying said sequence (eg, using PCR); and
(c) optionally inserting the sequence into an expression vector or a host cell genome for expression of the encoded antibody or domain.

In an example, an expression vector herein is a CHO cell expression vector for use in a CHO host cell. In an example, an expression vector herein is a HEK293 cell expression vector for use in a HEK293 host cell.

52. A method obtaining an antibody-producing cell line for the production of antibodies that specifically bind to an antigen, the method comprising inserting VH and VL-encoding nucleotide sequences into the genome of a host cell (eg, a CHO, HEK, MEF, COS or HeLa cell), wherein
(a) each VH sequence is operably inserted 5' of an antibody heavy chain constant region (eg, a human constant region) for expression of heavy chains by the cell comprising VH and C domains;
(b) each VL sequence is operably inserted 5' of an antibody light chain constant region (eg, a human constant region) for expression of light chains by the cell comprising VL and C domains;
(c) wherein the expressed heavy chains are capable of pairing with the light chains to produce heavy-light chain pairs, each pair comprising a VH/VL binding site that is capable of binding to an antigen; and
(d) wherein VH and VL-encoding nucleotide sequences are the sequences of VH and VL-encoding nucleotide sequences of one or more cells of Statement 41 or 47.

Optionally, the host cell is a HEK293 cell, eg, HEK293T or S cell, COS-1 or COS-7 cell.

53. The method of Statement 52, comprising inserting first and second VH-encoding nucleotide sequences into the cell genome, wherein
(a) the first VH (when expressed as part of first heavy chains) is capable of pairing with the VL (when expressed as part of light chains) to form a first VH/VL binding site that is capable of specifically binding to a first antigen;
(b) the second VH (when expressed as part of second heavy chains) is capable of pairing with the VL (when expressed as part of light chains) to form a second VH/VL binding site that is capable of specifically binding to a second antigen; and
(c) the first and second antigens are different;
   wherein the first and second heavy chains and the light chains are capable of pairing to produce bi-specific 4-chain antibodies comprising a first heavy chain-light chain pair and a second heavy chain-light chain pair, wherein
(d) the heavy chain of the first heavy chain-light chain pair comprises the first VH; and
(e) the heavy chain of the second heavy chain-light chain pair comprises the second VH domain.

54. A method of producing bi-specific 4- chain antibodies wherein the bi-specific antibodies comprise a respective binding site for first and second antigens, wherein the antigens are different, each antibody comprising a first heavy chain-light chain pair and a second heavy chain-light chain pair, wherein
(a) the heavy chain of the first heavy chain-light chain pair comprises a first VH, wherein the first VH (when expressed as part of first heavy chains) is capable of pairing with a VL (when expressed as part of light chains) to form a first VH/VL binding site that is capable of specifically binding to the first antigen; and
(b) the heavy chain of the second heavy chain-light chain pair comprises a second VH, wherein the second VH (when expressed as part of second heavy chains) is capable of pairing with said VL (when expressed as part of light chains) to form a second VH/VL binding site that is capable of specifically binding to the second antigen;
   the method comprising
(c) producing a first cell line that is capable of expressing the first heavy chains by (i) inserting into a first cell genome a nucleotide sequence encoding said first VH, wherein the VH sequence is operably inserted 5' of an antibody heavy chain constant region (eg, a human constant region) for expression of first heavy chains comprising first VH and C domains; and (ii) culturing the cell to produce a first cell line that expresses first heavy chains;
(d) producing a second cell line that is capable of expressing the second heavy chains by (i) inserting into a second cell genome a nucleotide sequence encoding said second VH, wherein the VH sequence is operably inserted 5' of an antibody heavy chain constant region (eg, a human constant region) for expression of second heavy chains comprising second VH and C domains; and (ii) culturing the cell to produce a second cell line that expresses second heavy chains;
(e) expressing first heavy chains from the first cell line and second heavy chains from the second cell line and mixing together first heavy chains comprising said first VH, second heavy chains comprising said second VH and light chains comprising said VL, whereby (iii) first heavy chains pair with light chains to produce said first heavy chain-light chain pairs, (iv) second heavy chains pair with light chains to produce said second heavy chain-light chain pairs; and (v) the first heavy chain-light chain pairs pair with the second heavy chain-light chain pairs, thereby producing said bi-specific antibodies
wherein VH and/or VL-encoding nucleotide sequences are the sequences of VH and/or VL-encoding nucleotide sequences of one or more cells of Statement 41 or 47.

Optionally, the first heavy chains and second heavy chains have first and second different pl values as described herein, wherein the method further comprises co-expressing the chains to produce a mixture and using pH change of the mixture to separate (eg, elute from a matrix) first chains from second chains, thereby producing a population of first chains that is separate from a population of second chains; and combining the first and second chains with the light chains to produce the bispecific antibodies.

The invention further provides an antibody (eg, a bispecific antibody) obtained or obtainable by a method of the invention. Optionally, the antibody is for treating or preventing a disease or condition in a human or animal subject.

55. The method of Statement 54, comprising
(a) producing a third cell line that is capable of expressing the light chains by (i) inserting into a third cell genome a nucleotide sequence encoding said VL, wherein the VL sequence is operably inserted 5' of an antibody light chain constant region (eg, a human constant region) for expression of light chains comprising the VL and C domains; and (ii) culturing the cell to produce a third cell line that expresses the light chains;
(b) expressing the light chains from the third cell line, wherein the light chains are the light chains of step (e) of Statement 54.

56. The method of Statement 54, comprising
(a) inserting into the genome of the first and/or second cell or cell lines a nucleotide sequence encoding said VL, wherein the VL sequence is operably inserted 5' of an antibody light chain constant region (eg, a human constant region) for expression of light chains comprising the VL and C domains; and
(b) expressing the light chains from the cell lines comprising the VH sequence, wherein the light chains are the light chains of step (e) of Statement 54.

57. The method of Statement 54, 55 or 56, wherein said mixing of chains is carried out by co-culturing the cell lines.

58. The method of Statement 54, 55 or 56, wherein said mixing of chains is carried out by isolating chains expressed by the cell lines and mixing the isolated chains together (eg, in a container that does not comprise said cells).

Said isolating can be performed, for example, using differential pl of chains as described herein.

59. A method of producing bi-specific 4-chain antibodies wherein the bi-specific antibodies comprise a respective binding site for first and second antigens, wherein the antigens are different, each antibody comprising a first heavy chain-light chain pair and a second heavy chain-light chain pair, wherein
(a) the heavy chain of the first heavy chain-light chain pair comprises a first VH, wherein the first VH (when expressed as part of first heavy chains) is capable of pairing with a VL (when expressed as part of light chains) to form a first VH/VL binding site that is capable of specifically binding to the first antigen; and
(b) the heavy chain of the second heavy chain-light chain pair comprises a second VH, wherein the second VH (when expressed as part of second heavy chains) is capable of pairing with said VL (when expressed as part of light chains) to form a second VH/VL binding site that is capable of specifically binding to the second antigen;
   the method comprising
(c) mixing together first heavy chains comprising said first VH, second heavy chains comprising said second VH and light chains comprising said VL, whereby (i) first heavy chains pair with light chains to produce said first heavy chain-light chain pairs, (ii) second heavy chains pair with light chains to produce said second heavy chain-light chain pairs; and (iii) the first heavy chain-light chain pairs pair with the second heavy chain-light chain pairs, thereby producing said bi-specific antibodies
wherein VH and/or VL are encoded by VH and/or VL-encoding nucleotide sequences, wherein the nucleotide sequences are the sequences of VH and/or VL-encoding nucleotide sequences of one or more cells of Statement 41 or 47.

60. The method of any one of Statements 54 to 59, wherein each 4-chain antibody comprises mutated heavy chain constant regions to promote pairing of the heavy chains of the first and second heavy-light pairs; optionally wherein the mutations are knob-in-hole mutations or charge pair mutations.

For example, expression vectors can be provided into which a VH-encoding sequence is inserted 5' in in operably connection with a mutated human heavy chain constant region-encoding nucleotide sequence, wherein the mutation is for knob-in-hole pairing with another heavy chain or for charge pairing with another heavy chain. Thus, the VH of the heavy chain of the first heavy-light pair can be inserted into such a vector 5' of a first mutated heavy chain constant region, and the VH of the heavy chain of the second heavy-light pair can be inserted into such a vector (the same or a different vector) 5' or a second mutated heavy chain constant region, wherein the first and second heavy chains are expressible from the vector(s) and can pair using the knob-in-hole or charge pair mutations. Alternatively, the VH-encoding sequences are inserted 5' of respective constant regions in the genome of the same or different host cells for expression of said heavy chains.

61. A method of producing a pharmaceutical composition for treating or preventing a disease or condition (eg, a cancer or autoimmune disease) in a human or non-human animal subject, the method comprising
(a) expressing an antibody from a cell line produced by the method of Statement 52 or 53; and
(b) mixing the antibody with a diluent or excipient;
(c) optionally wherein the antibody is identical to, or comprises variable domains identical to, an antibody obtained by the method of any one of Statements 54 to 60.

62. A method of producing a pharmaceutical composition for treating or preventing a disease or condition (eg, a cancer or autoimmune disease) in a human or non-human animal subject, the method comprising mixing a bi-specific antibody with a diluent or excipient, wherein the antibody is identical to, or comprises variable domains identical to, an antibody obtained by the method of any one of Statements 54 to 60.

63. A method of producing a cell of any one of Statements 1 to 28 or vertebrate of any one of Statements 29 to 39, the method comprising
(a) obtaining a nucleic acid (eg, DNA or cDNA) comprising a rearranged antibody variable region encoding a rearranged antibody V domain; and
(b) inserting the variable region or a copy thereof into the genome of a cell, whereby the variable region is comprised by an antibody locus in said genome,
   wherein the locus comprises (in 5' to 3' direction)
(c) an antibody locus intronic enhancer;
(d) said rearranged antibody variable region; and
(e) an antibody constant region encoding an antibody C domain;
   wherein the locus is operable to express an antibody chain comprising (in N- to C-terminal direction) said rearranged V domain and said C domain; and
(f) optionally, wherein the cell is a non-human vertebrate ES cell or an iPS cell, the method further comprising generating the non-human vertebrate of any one of Statements 29 to 39 from the cell.

Optionally, the cell is a pluripotent, ES or iPS cell and the cell is not capable of generating a human or a human embryo. For example, the cell is not a human ES or iPS or pluripotent stem cell.

Optionally, the cell is a non-human vertebrate (eg, rodent, mouse or rat) cell, eg, ES or iPS cell.

Standard techniques will be known by, and readily available to, the skilled addressee for producing transgenic non-human vertebrates (eg, mice and rats) using the ES or iPS cells of the invention. For example, one or more ES cells of the invention can be introduced into a recipient blastocyst or pre-morula embryo before implantation into a female non-human vertebrate (eg, a pseudopregnant mouse or rat when the ES and blastocyst/pre-morula embryo are mouse or rat respectively). Optionally, said other cells, blastocyst or pre-morula embryo comprise a RAG knock-out (eg, RAG-1 and/or RAG-2 knock-out). This is useful to ensure that all antibody chains, such as chains produced by cells and loci of the invention, are contributed 100% in the non-human vertebrate by the loci of the invention and not the blastocyst or pre-morula embryo cells. Additionally or alternatively, endogenous IgH expression is knocked-out or inactivated, wherein the non-human vertebrate of the invention is capable of expressing antibody heavy chains comprising human variable domains and human or non-human vertebrate constant domains (eg, from a transgenic IgH locus comprised by cells of the vertebrate).

Optionally, the nucleic acid of step (a) comprises (in 5' to 3' direction) said rearranged antibody variable region and a constant region encoding one or more antibody C domains (eg, a Cκ, Cλ or heavy chain Fc). Optionally, the nucleic acid further comprises (5' of the variable region) a promoter operable for promoting transcription of the variable region, and a nucleotide sequence encoding a variable domain signal peptide, wherein the nucleic acid encodes a signal peptide-V domain - C domain.

Optionally, the locus is an antibody light chain locus (first locus), wherein the locus comprises (in 5' to 3' direction)
(g) A light chain intronic enhancer;
(h) A rearranged antibody variable region encoding a rearranged antibody V domain; and
(i) An antibody light chain constant region encoding a light chain C domain;
wherein the locus is operable in the cell to express an antibody chain comprising (in N- to C-terminal direction) said rearranged V domain and said C domain.

Optionally, the locus is an antibody light chain locus (first locus), wherein the locus comprises (in 5' to 3' direction) a light chain intronic enhancer and a nucleotide sequence encoding a rearranged light chain, wherein the locus is operable in the cell to express the light chain.

Optionally, the cell is a rodent (eg, a mouse or rata cell) and the locus is an antibody kappa light chain locus (first locus), wherein the locus comprises (in 5' to 3' direction) a mouse or rat Eiκ intronic enhancer and a nucleotide sequence encoding a rearranged human light chain, wherein the locus is operable in the cell to express the light chain.

In an example of any cell, vertebrate, population, composition or method of the invention, the V domain is a Vκ (eg, human Vκ) and the C domain is a Cκ (eg, a human Cκ). In an example, the V domain is a Vλ (eg, human Vλ) and the C domain is a Cλ (eg, a human Cλ). For example, the nucleic acid encodes a rearranged light chain of an antibody (eg, wherein the antibody specifically binds to a predetermined antigen or epitope).

64. The method of Statement 63, wherein the variable domain is a VL (eg, a human Vλ or Vκ) and the C domain is CL (eg, a human Cλ or Cκ).

For example, the variable domain is a human Vλ and the C domain is a human Cλ. For example, the variable domain is a human Vκ and the C domain is a human Cκ.

65. The method of Statement 63 or 64, wherein said inserting is carried out using homologous recombination or site-specific recombination (eg, using lox sites) with chromosomal DNA of the cell.

66. The method of any one of Statements 63 to 65, comprising
obtaining a transgene comprising (in 5' to 3' direction)
(a) said intronic enhancer (eg, an Eiκ, such as a mouse or rat Eiκ);
(b) said rearranged antibody variable region (eg, a human VL-encoding variable region); and
(c) said antibody constant region (eg, a human CL-encoding region); and
Inserting said transgene into the genome of the cell, whereby said antibody locus is comprised by the cell.

For example, the insertion is at or adjacent the Rosa26 locus of the cell, or randomly made into the genome.

67. The method of any one of Statements 63 to 65, comprising inserting said variable region between
(a) the intronic enhancer and the CL of a light chain locus of the cell genome; or
(b) the intronic enhancer (Eµ) and the Cµ of a heavy chain locus of the cell genome.

In any insertion herein, nucleotide sequence of the recipient nucleic acid (eg, cell chromosomal DNA) may be deleted simultaneously or sequentially with the deletion. Alternatively, insertion is without deletion of recipient nucleic acid sequence.

68. The method of any one of Statements 63 to 67, wherein the variable region encodes a V domain (eg, a VL domain) of an antibody that specifically binds to a predetermined antigen.

69. The method of Statement 68, wherein the step of obtaining the nucleic acid comprising the rearranged antibody variable region as recited in Statement 63 comprises
(a) obtaining a further cell (eg, a hybridoma or B-cell) that expresses said antibody recited in Statement 68;
(b) obtaining or copying (eg, using PCR)
   i. a DNA (eg, a chromosomal DNA or a cDNA) sequence of the further cell that comprises a rearranged variable region sequence encoding the V domain; or
   ii. a RNA sequence (eg, a mRNA sequence sequence) of the further cell that comprises a rearranged variable region sequence encoding the V domain, and creating a DNA copy of said RNA sequence;
thereby obtaining said nucleic acid.

In an example, RNA may be used to produce a cDNA copy thereof, the cDNA being used in the method of the invention.

70. The method of any one of Statement 63 to 69, comprising culturing the resultant cell to produce a plurality of cells, wherein each cell is according to any one of Statements 1 to 28.

Optionally, said plurality comprise at least 100, 10³, 10⁴, 10⁵, 10⁶, 10⁷, 10⁸, 10⁹, 10¹⁰, 10¹¹ or 10¹² cells, wherein each cell is according to the invention (optionally wherein each cell is identical to the other cells or the cells comprise the same first locus of the invention).

71. A non-human vertebrate (eg, rodent, rat or mouse) blastocyst or pre-morula embryo implanted with an ES or iPS cell according to any one of Statements 1 to 28 or obtained according to the method of any one of Statements 63 to 70.

Optionally, said blastocyst or pre-morula embryo comprises a RAG knock-out (eg, RAG-1 and/or RAG-2 knock-out). This is useful to ensure that all antibody chains in non-human vertebrates grown from the blastocyst or pre-morula embryo are contributed 100% in the non-human vertebrate by the loci if the invention and not the blastocyst or pre-morula embryo cells. In an example, the recipient blastocyst or pre-morula is of a mouse strain, such as C57BL/6 and optionally the ES or iPS cells are of a 129, 129 hybrid, F1H4 or AB2.1 strain.

72. A nucleic acid (eg, a DNA) comprising a transgene for use in the method of Statement 66, the transgene comprising (in 5' to 3' direction)
(a) said intronic enhancer;
(b) said rearranged antibody variable region; and
(c) said antibody constant region.

73. A nucleic acid comprising a rearranged variable region sequence encoding a rearranged V domain for use in the method of Statement 67, the nucleic acid comprising said rearranged variable region sequence flanked
(a) 5' by a homology arm for hybridising to a first nucleotide sequence of the cell genome, and/or
(b) 3' by a homology arm for hybridising to a second nucleotide sequence of the cell genome;
   Wherein
(c) the 5' homology arm is homologous to a sequence of an antibody light chain (eg, kappa) locus of the cell 5' of the CL region thereof, and the 3' homology arm is homologous to a sequence of said locus 3' of the intronic enhancer (eg, Eiκ) of the locus, whereby homologous recombination between the homology arms and the genome of the cell is capable of inserting the variable region sequence, thereby producing a locus comprising (in 5 to 3' direction) an intronic enhancer, said variable region and a CL region; or
(d) the 5' homology arm is homologous to a sequence of a heavy chain antibody locus of the cell 5' of the Cµ region thereof, and the 3' homology arm is homologous to a sequence of said locus 3' of the intronic enhancer (Eµ) of the locus, whereby homologous recombination between the homology arms and the genome of the cell is capable of inserting the variable region sequence, thereby producing a locus comprising (in 5 to 3' direction) an intronic enhancer, said variable region and a Cµ region.

74. The nucleic acid of Statement 73, wherein nucleic acid or the 5' homology arm comprises (in 5' to 3' direction)
(a) a promoter operable for promoting transcription of the variable region;
(b) a nucleotide sequence encoding a variable domain signal peptide; and
(c) Optionally a said intronic enhancer sequence,
Wherein when the variable region sequence is inserted into the cell genome, the variable region is inserted in operable connection 3' of the promoter and signal peptide-encoding sequence for expression of an antibody chain comprising (in N- to C-terminal direction) a singal sequence-V domain-C domain.

The invention further provides a composition (eg, a pharmaceutical composition or a composition for medical use) comprising an antibody, bispecific antibody, antibody chain (eg, light chain), VL or nucleotide sequence thereof obtained or obtainable by a method of the invention as disclosed herein; optionally wherein the composition comprises a diluent, excipient or carrier, optionally wherein the composition is contained in an IV container (eg, and IV bag) or a container connected to an IV syringe. When the composition is a pharmaceutical composition or a composition for medical use, the diluent, excipient or carrier is pharmaceutically acceptable. "Pharmaceutically acceptable" refers to approved or approvable by a regulatory agency of the USA Federal or a state government or listed in the U.S. Pharmacopeia or other generally recognized pharmacopeia for use in animals, including humans. A "pharmaceutically acceptable carrier, excipient, or adjuvant" refers to a carrier, excipient, or adjuvant that can be administered to a subject, together with an agent, e.g., any antibody, VL or antibody chain described herein, and which does not destroy the pharmacological activity thereof and is nontoxic when administered in doses sufficient to deliver a therapeutic amount of the agent.

The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "e.g." is derived from the Latin exempli gratia, and is used herein to indicate a non-limiting example. Thus, the abbreviation "e.g." is synonymous with the term "for example."

Definitions of common terms in cell biology and molecular biology can be found in "The Merck Manual of Diagnosis and Therapy", 19th Edition, published by Merck Research Laboratories, 2006 (ISBN 0-911910-19-0); Robert S. Porter et al. (eds.), The Encyclopedia of Molecular Biology, published by Blackwell Science Ltd., 1994 (ISBN 0-632-02182-9); Benjamin Lewin, Genes X, published by Jones & Bartlett Publishing, 2009 (ISBN-10: 0763766321); Kendrew et al. (eds.), , Molecular Biology and Biotechnology: a Comprehensive Desk Reference, published by VCH Publishers, Inc., 1995 (ISBN 1-56081-569-8) and Current Protocols in Protein Sciences 2009, Wiley Intersciences, Coligan et al., eds.

Unless otherwise stated, the present invention was performed using standard procedures, as described, for example in Sambrook et al., Molecular Cloning: A Laboratory Manual (4 ed.), Cold Spring Harbor Laboratory Press, Cold Spring Harbor, N.Y., USA (2012); Davis et al., Basic Methods in Molecular Biology, Elsevier Science Publishing, Inc., New York, USA (1995); or Methods in Enzymology: Guide to Molecular Cloning Techniques Vol.152, S. L. Berger and A. R. Kimmel Eds., Academic Press Inc., San Diego, USA (1987); Current Protocols in Protein Science (CPPS) (John E. Coligan, et. al., ed., John Wiley and Sons, Inc.), Current Protocols in Cell Biology (CPCB) (Juan S. Bonifacino et. al. ed., John Wiley and Sons, Inc.), and Culture of Animal Cells: A Manual of Basic Technique by R. Ian Freshney, Publisher: Wiley-Liss; 5th edition (2005), Animal Cell Culture Methods (Methods in Cell Biology, Vol. 57, Jennie P. Mather and David Barnes editors, Academic Press, 1st edition, 1998) which are all incorporated by reference herein in their entireties.

Other terms are defined herein within the description of the various aspects of the invention.

All patents and other publications; including literature references, issued patents, published patent applications, and co-pending patent applications (including the US counterparts of any of these); cited throughout this application are expressly incorporated herein by reference for the purpose of describing and disclosing, for example, the methodologies described in such publications that might be used in connection with the technology described herein. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments may perform functions in a different order, or functions may be performed substantially concurrently. The teachings of the disclosure provided herein can be applied to other procedures or methods as appropriate. The various embodiments described herein can be combined to provide further embodiments. Aspects of the disclosure can be modified, if necessary, to employ the compositions, functions and concepts of the above references and application to provide yet further embodiments of the disclosure. Moreover, due to biological functional equivalency considerations, some changes can be made in protein structure without affecting the biological or chemical action in kind or amount. These and other changes can be made to the disclosure in light of the detailed description. All such modifications are intended to be included within the scope of the appended claims.

Specific elements of any of the foregoing embodiments can be combined or substituted for elements in other embodiments. Furthermore, while advantages associated with certain embodiments of the disclosure have been described in the context of these embodiments, other embodiments may also exhibit such advantages, and not all embodiments need necessarily exhibit such advantages to fall within the scope of the disclosure.

It will be understood that particular configurations, aspects, examples, embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains. All publications and patent applications (including US equivalents) are herein incorporated by reference to the same extent as if each individual publication or patent application was specifically and individually indicated to be incorporated by reference. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Any part of this disclosure may be read in combination with any other part of the disclosure, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. All such similar substitutes and modifications apparent to those skilled in the art are deemed to be within the spirit, scope and concept of the invention as defined by the appended claims.

The present invention is described in more detail in the following non limiting Examples.

### EXAMPLES

### Example 1. Construction of targeting vectors for mouse ES cell targeting

### 1.1 pUC57_N128L_KI vector DNA synthesis

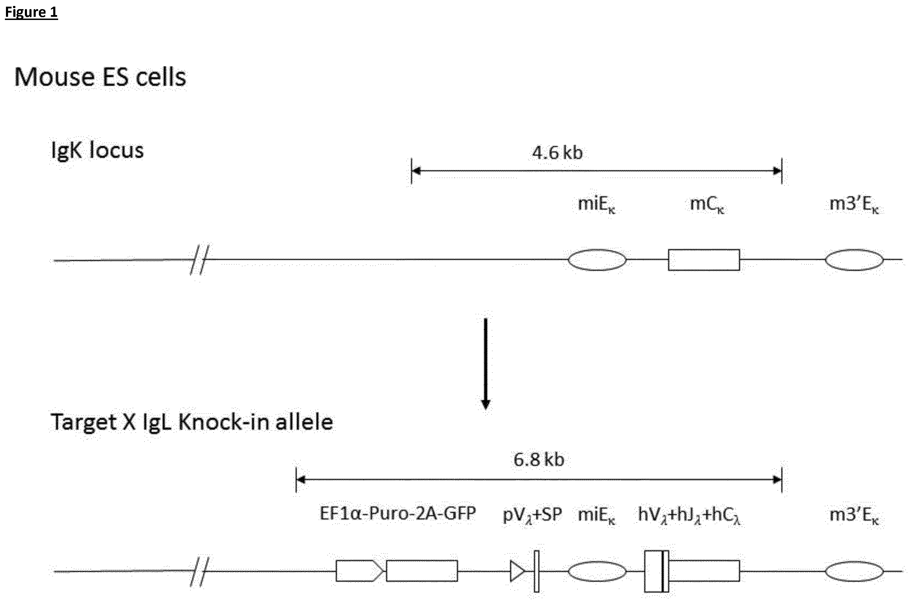

N128L is a human λ light chain rearranged from human IGLV3-21*01 and IGLJ3*02 (VL domain sequence is given below). A DNA fragment containing wildtype human IGLV3-21*01 promoter (pV_{λ}) and signal peptide (SP), mouse intronic κ enhancer (miE_{κ}), and N128L light chain variable region and constant region was synthesized by Genscript. A 1-kb fragment between Y_{κ} and J_{K} region in the kappa locus, and a 1-kb fragment between mouse κ constant region (C_{κ}) and mouse 3' κ enhancer (m3'E_{κ}) were used as homology arms for targeting the N128L knock-in cassette. After successful targeting, kappa allele DNA including mouse C_{κ} was replaced by the N128L knock-in (Figure 1).

### 1.2 pUC57_N128L_EF1α_Puro_2A_EGFP_SV40pA targeting vector

EF1α_Puro_2A_EGFP_SV40pA vector was digested with Notl and Ascl. A 3.6 kb fragment was purified using QIAquick^{™} PCR Purification Ki (QIAGEN) by the method described in the attached instruction manual. pUC57_N128L_KI vector was digested with Notl and Ascl. A 7.9 kb fragment was purified QIAquick PCR Purification Kit (QIAGEN) by the method described in the attached instruction manual. The Notl-Ascl-digested EF1α_Puro_2A_EGFP_SV40pA and pUC57_N128L_KI fragments were ligated using T4 ligase (New England Biolabs) according to the method described in the attached instruction manual. *E.coli* DH10B strain (ElectroMax^{™} DH10B (Invitrogen)) was transformed with the ligation solution. Respective plasmid DNAs were isolated from the obtained ampicillin resistant clones using QIAprep^{™} Spin Miniprep Kit (QIAGEN). The resulting respective ampicillin resistant transformants were confirmed to have the insertion by Sanger sequencing.

### 1.3 Preparation of plasmid DNA for mouse ES cell targeting

For pUC57_N128L_EF1α_Puro_2A_EGFP_SV40pA targeting vector, plasmid DNA was isolated from the sequencing verified clones using QlAprep Maxi plus Kit^{™} (QIAGEN) by the method described in the attached instruction manual. Respective plasmid DNAs were confirmed to have the insertion by Sanger sequencing.

### Example 2. Generation of N128L knock-in mouse ES cell lines

### 2.1 Preparation of mouse ES cells

Cells from two independent mouse embryonic stem cell (ES) lines were expanded on STO feeder plates for electroporation. ES cells were fed with fresh M15 media until they reached 80% to 85% confluence under microscope. The knock-in construct was introduced using electroporation.

ES cells were cultured in M15 media supplemented with Puromycin (1 µg/mL). Seven days after electroporation, Puromycin resistant colonies were big enough for picking.

### 2.2 Microinjection of targeted mouse ES cell clones

Targeted ES cells were injected to C57BL6 strain blastocysts. After injection, blastocysts were transferred to the uterus of C57BL6 strain F1 hybrid females mated with vasectomised males 3 days before injection. The chimaeric embryos were allowed to develop to term in the pseudo-pregnant recipients, which developed into pups comprising the light chain knock-in.

### Example 3: Common Light Chain Mice

Eight chimaera mice with common light chain knock-in allele and a functional unrearranged human VH region in an IgH locus were immunized with human Target X.

The knock-in encodes for the following N128L VL (in N- to C-terminal direction):-

SYVLTQPPSVSVAPGETARITCGGDNIGRKSVYWYQQKSGQAPVLVIYYDSDRPSGIPERFSGSNSGNTATLTISRVE
AGDEADYYCQVWDGSSDHWVFGGGTKLTVL

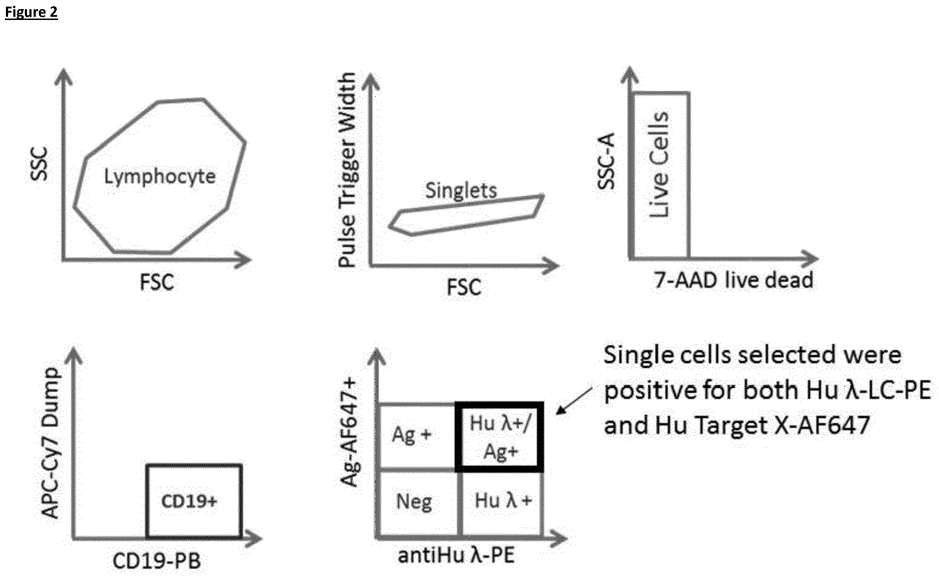

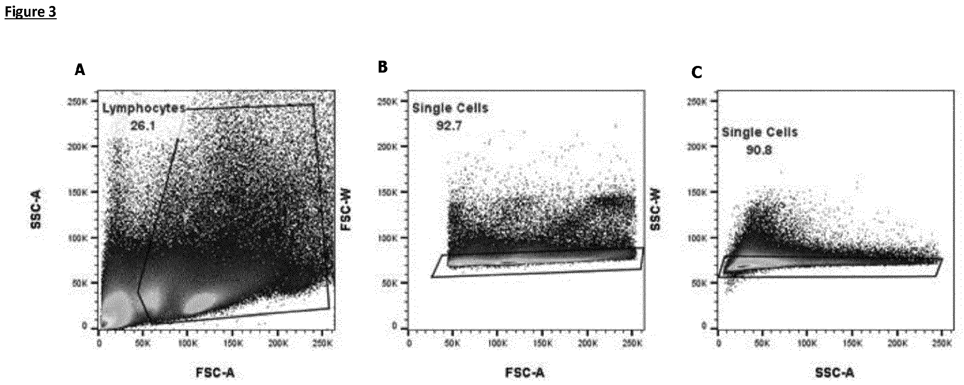

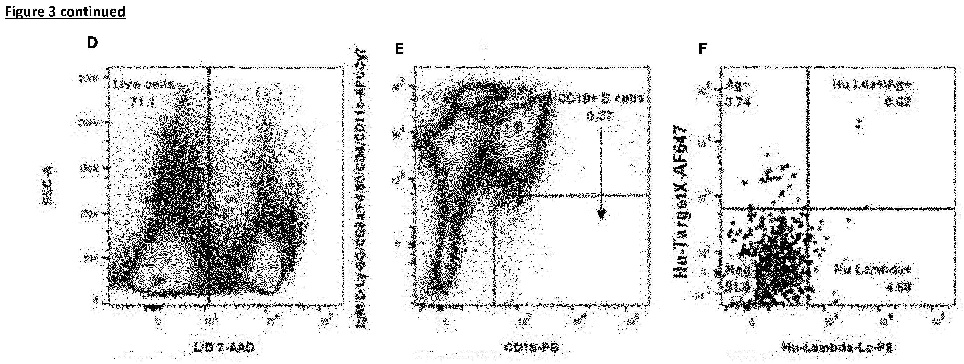

After immunization, sorting was performed on spleen B cells using a monoclonal antibody against human λ light chain labelled with PE and human Target X labelled with AF647 (Figs 2 & 3). B cells that were positive for both PE and AF647 were sorted as single cell into individual wells on a 96-well plate. Single cell NGS libraries were constructed using standard protocol and subjected to NGS sequencing.

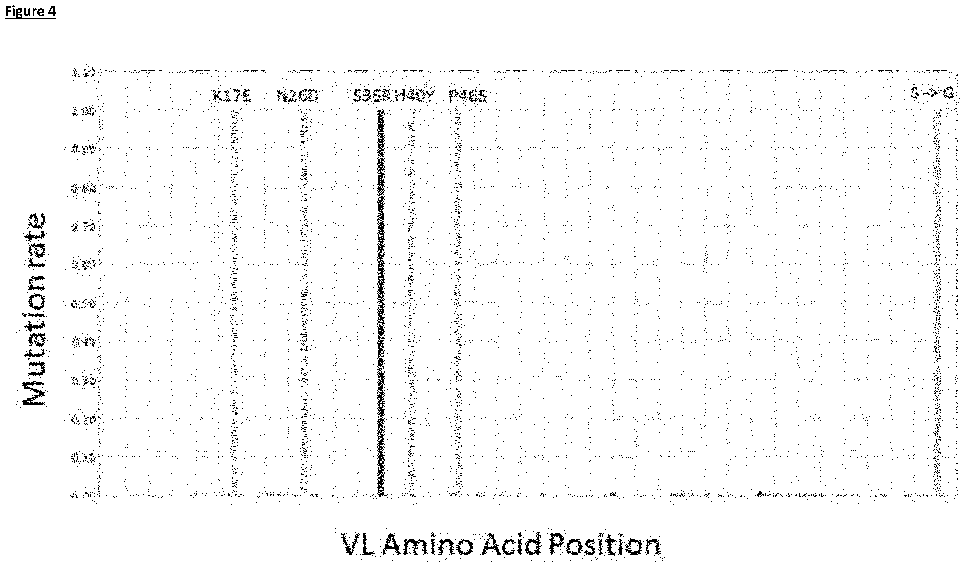

After subtraction of sequences from a PCR control included in our experiment, we found 346 clones, all of which comprised the expected λ light chain combination derived from IGLV3-21*01 and IGLJ3*02. Clustering the light chain sequences with that of the knock-in light chain allele demonstrated that 339 out of 346 clones were unmutated, with the other 7 clones having one or more mutations (Fig 4).

**Table 1: Common L Chain Frequencies**

| **Number of Clones (out of 346 total)** | **Percent Identity to predetermined human A light chain rearranged from human IGLV3-21*01 and IGLJ3*02** |
|---|---|
| 339 | 100.00% |
| 1 | 97.23% |
| 1 | 98.15% |
| 1 | 99.08% |
| 4 | 99.69% |

Compared to sequences that are a perfect (ie, 100%) match for the inserted light chain sequence, ones that do not completely match have lower confidence scores (81.6% confidence score (for the average of the sequences assigned a 100% match) versus 31.7% confidence score (for the average of the 7 other sequences), 100% represents the highest confidence, 0% represents the lowest confidence). Thus, we found that of all the VLs that were based on the human IGLV3-21*01 and IGLJ3*02 combination, 98% comprised the identical input common light chain V sequence.

After bioinformatics analysis, desirably the human heavy chain V domain sequence repertoire of the clones was found to be diverse. There were no dominant human V_{H}, D_{H} or J_{H} usage identified by the bioinformatics analysis. This matches the bulk NGS analysis of variable regions of heavy chains in spleen B cells from naive chimera mice (data not shown).

Human VH gene segment usage comprised the following:
IGHV1-3*01, IGHV1-8*01, IGHV1-18*01, IGHV1-46*03, IGHV3-7*01, IGHV3-9*01, IGHV3-11*01, IGHV3-15*01, IGHV3-20*d01, IGHV3-21*03, IGHV3-23*04, IGHV3-30*18, IGHV3-33*01, IGHV3-48*02, IGHV3-53*01, IGHV4-4*02, IGHV4-31*03, IGHV4-34*01, IGHV4-39*01, IGHV4-59*01, IGHV4-61*01, IGHV5-51*01, IGHV6-1*01 and IGHV7-4-1*01.

### Example 4: Construction of Common Light Chain Targeting Vector For Mouse ES Cell Targeting

Further common light chain mice were made as follows.

### Generation of common light chain targeting vector

A DNA fragment containing wild-type human V_{λ} promoter (pV_{λ}) and signal peptide (SP), mouse intronic κ enhancer (miE_{κ}), and nucleotide sequence encoding a human light chain variable region and constant region was synthesized. The light chain variable domain has specificity for a target (Target Z) other than Target Y used below. A 1kb fragment between Y_{κ} and J_{κ} (1-5), and a 1kb fragment between mouse κ constant region (C_{κ}) and mouse 3' κ enhancer (m3'E_{κ}) were used as homology arms for targeting the common light chain knock-in cassette. After successful targeting, the human J_{κ} (1-5) and mouse C_{κ} will be replaced by common light chain knock-in cassette on one or both alleles.

### Generation of common light chain targeted mouse ES cells

Cells from two independent mouse embryonic stem cell (ES) lines were expanded on STO feeder plates for electroporation. ES cells were fed with fresh M15 media until they reached 80% to 85% confluence under microscope.

Two hours before electroporation, ES cells were fed with fresh M15 media. Before electroporation, ES cells were washed twice with PBS, and dissociated from plates by adding trypsin. After incubation at 37°C for 20 minutes, the trypsin was inactivated by adding same volume of M15 media. ES cells were dissociated to a single cell suspension by repeat pipetting. ES cells were pelleted by centrifuging at 1,100 rpm for 4 minutes, and re-suspended in electroporation buffer (Lonza) by the method described in the attached instruction manual.

Electroporation were performed using Lonza Amaxa^{™} machine using preset mouse ES cell protocol. After electroporation, ES cells from each electroporation were re-suspended in fresh M15 media, and plated onto one 10 cm feeder plate.

ES cells were fed with M15 media for 3 days after electroporation. ES cells from each electroporation were passaged 1:3 to three 10 cm feeder plates. Two days after passage, ES cells were cultured in M15 media supplemented with Puromycin (1 µg/mL). Seven days after electroporation, Puromycin resistant colonies were big enough for picking.

Puromycin resistant colonies on 10 cm STO feeder plates were fed with fresh M15 media 2 hours before picking. 50 µl of trypsin was added to each well of a 96-well round bottom plate. Colonies on 10 cm STO feeder plates twice with PBS buffer. 10 ml of PBS buffer was added to each 10cm plate.

Single colonies were picked from the feeder plates using a Gilson pipette, and transferred into the well with trypsin of 96-well plate (1 colony/well). After completing a plate (96 colonies picked), the trypsin plate was incubated at 37°C for 30 minutes. After that, 150 µl of fresh M15 media was added to each well. Colonies were dissociated by repeat pipetting. Single cell suspension was transferred to a 96-well feeder plate and cultured in a TC incubator at 37°C. ES cells were fed with fresh M15 media until they reached 80% to 85% confluence under microscope.

### Passaging common light chain mouse ES cells

Two hours before passaging, ES cells were fed with fresh M15 media. Before passaging, ES cells were washed twice with PBS buffer, and dissociated from plates by adding trypsin. After incubation at 37°C for 20 minutes, the trypsin was inactivated by adding same volume of M15 media. ES cells were dissociated to a single cell suspension by repeat pipetting.

Single ES cell suspension were split 1:4 to four 96 well STO feeder plates. ES cells were fed with fresh M15 media until they reached 80% to 85% confluence under microscope.

### Freezing ES cells on 96 well plate

Two hours before freezing, ES cells were fed with fresh M15 media. Before freezing, ES cells were washed twice with PBS buffer, and dissociated from plates by adding trypsin. After incubation at 37°C for 20 minutes, the trypsin was inactivated by adding same volume of 2X freezing media (60% DMEM, 20% FBS, 20% DMSO, freshly prepared). ES cells were dissociated to a single cell suspension by repeat pipetting. 96 well plates were sealed and placed in a polystyrene box inside a -80°C freezer to facilitate a very slow freezing process.

### Genomic DNA extraction

ES cells were washed twice with PBS buffer, and 50 µL of ES cell Lysis Buffer (10 mM Tris-HCl, pH 7.5, 10 mM EDTA, 0.5% SDS, 10 mM NaCl) with Proteinase K (1 mg/mL) was added to each well of the 96 well plates. The plates were sealed, wrapped with wet tissues, and placed into a lunch box. The lunch box was incubated at 55 °C overnight.

The second day, 100 µL ice-cold Ethanol/NaCl mix (1.5 mL 5M NaCl added to 98.5 mL 100% ethanol) was added to each well of the 96 well plates. The plates were at room temperature for about 30 minutes. After incubation, the plates were centrifuged at 3,000 rpm for 20 minutes. The plates were inverted to decant liquid, and dried on paper towels to remove access liquid. The plates were washed twice by adding 150 µL cold 70% ethanol, and dried completely on paper towels. After the DNA pellets were dried, 30 µL of TE (pH 8.0) buffer was added and DNA was resuspended by incubation at 55 °C for 30 minutes.

### Thawing and expansion of common light chain mouse ES cell clones

Frozen 96 well plates were taken out of the -80°C freezer, and thawed in a 37°C incubator until every well of the plates became transparent. 2 mL fresh M15 media was added to each well of 24 well STO feeder plates. All the contents from the desired wells of the 96 well plates were transferred to a well of the 24 well plates. After all the targeted clones were transferred to a 24 well plate, the plate was cultured in a TC incubator at 37°C. ES cells were fed with fresh M15 media until they reached 80% to 85% confluence under microscope. Once they became confluent, they were 1:1 passaged to 6-well STO feeder plates and frozen down for microinjection.

### Microinjection of common light chain mouse ES cell clones

Mouse ES cells were thawed and expanded before injection. Two hours before injection, ES cells were fed with fresh M15 media. Before injection, ES cells were washed twice with PBS buffer, and dissociated from plates by adding trypsin. After incubation at 37°C for 20 minutes, the trypsin was inactivated by adding same volume of fresh M15 media. ES cells were dissociated to a single cell suspension by repeat pipetting, and put on ice for injection.

Targeted ES cells were injected to E3.5 Rag^{-/-} blastocysts. After injection, blastocysts were transferred to the uterus of CBA;C57BL6/J F1 hybrid females mated with vasectomised males 3 days before injection. The chimeric embryos were allowed to develop to term in the pseudo-pregnant recipients. The resulting mice comprised cells derived from the engineered ES cells, where the cell genomes comprised the human lambda light chain knock-in at one mouse kappa locus (the other kappa locus was inactivated by deletion in the J region) and an unrearranged human heavy chain variable region comprised by a homozygous IgH locus.

### Example 5: Production of Antibody To Target Y

### Immunization

Common light chain chimera mice were immunized with Target Y as described below. As the first immunization, 40 µg/head of Target Y, emulsified by CpG (Sigma) and Alum (1:1 antigen), was subcutaneously administered. Four weeks later, 5 µg/head of Target Y, emulsified by by CpG (Sigma) and Alum (1:1 antigen) was administered by intraperitoneal injection (first boost). One week later, four mice were sacrificed and the spleens were extracted. Four weeks after the first boost, 2 µg/head of Target Y, emulsified by by CpG (Sigma) and Alum (1:1 antigen) was administered by intraperitoneal injection (second boost) to the another four mice One week later these mice were sacrificed, and the spleens were extracted.

### Antigen specific B cell sorting

Harvested and processed spleen tissues were resuspended in 20 mL 3% FBS/PBS. Cells were pelleted at 300 g for 10 seconds at 40°C, and resuspended in 300 µl 3% FBS/PBS buffer.

20ul of Fc blocker was added for 10 seconds and stored at 4°C before use.

Alexa-647 labelled human Target Y stock solution (0.8 mg/mL) was diluted to 20 µg/mL and used at 1:40 dilution (final concentration 0.5 µg/mL) for staining.

A 100 µl staining cocktail containing CD19-PB, IgM-APC-Cy7, IgD-APC-Cy7, CD38-FITC, CD95-PE, Hu-Target Y - Alexa 647, was mixed and added to 300 µl spleen cell suspension. After incubation at 4°C for 20 minutes, 5 ml of 3% FBS/PBS buffer was added to the samples. Cells were pelleted at 300 g for 10 minutes, and resuspended in 400 µl of 3% FBS/PBS buffer and filter through 30 µm cell strainer. Five minutes before sorting, 8 µl of 7AAD (1:100 dilution) was added. The gating strategy for sorting is shown below.

### B-Cell Screening (BCT) and recovery of coupled antibody heavy and light chains for expression

BCT technology is generally described in WO2015/040401, which is incorporated herein by reference.

For RT, a mixture of 0.1 µL of a IgG constant region specific primer (10 µM), 0.1 µL of a IgK constant region specific primer (10 µM), 0.05 µL a IgL constant region specific primer (10 µM), 1 µL dNTP mix (10 mM), 2 µL of 5X First-Strand Buffer, 1 µL of 0.1 M DTT, 0.4 µL of RNaseOUT^{™} RNase Inhibitor (Invitrogen), 0.25 µL of Superscript^{™} III RT (200 units/µl, Invitrogen), and 1.1 µL of RNase-free water were added to each well to make a final volume of 10 µL. RT reactions were performed to generate 1^{st} strand cDNA.

For 1^{st} round PCR, a mixture of 12.5 µL of 2X Q5 master mix, 0.15 µL of a mixture of VH specific primers (10 µM), 0.1 µM of a mixture of VL specific primers (10 µM), 0.15 µL of a mixture of VK specific primers (10 µM), 0.1 µL of a IgG constant region specific primer (10 µM), 0.1 µL of a IgK constant region specific primer (10 µm), 0.1 µL of a IgL constant region specific primer (10µm), and 1.8 µL of RNase-free water was mixed with 10 µL of RT product in each well to make a final volume of 25 µL. PCR reactions were performed to amplify heavy chain and light chain variable regions.

For 2^{nd} round PCR, a mixture of 12.5 µL of 2X Q5 master mix, 0.2 µL of a VH nested PCR primer (10 µM), 0.2 µL of a VK nested PCR primer (10 µM), 0.2 µL of a VL nested PCR primer (10 µM), 0.2 µL of a IgG constant region nested PCR primer (10 µM), 0.2 µL of a IgK constant region nested PCR primer (10 µM), 0.2 µL of a IgL constant region nested PCR primer (10 µM), and 10.3 µL of RNase-free water was mixed with 1 µL of 1^{st} round PCR product in each well to make a final volume of 25 µL. PCR reactions were performed to amplify heavy chain and light chain variable regions.

For bridge PCR, a mixture of 15 µL of 2X Q5 master mix, 0.1 µL of Heavy Vector (100 ng/ µl), 0.05 µL of Kappa vector (100ng/µl), and 1 µL of HLP479/480 mix (10 µM each), and 12.85 µL of RNase-free water was mixed with 1 µL of 2^{nd} round PCR product in each well to make a final volume of 25 µL. PCR reactions were performed to amplify heavy chain and light chain variable regions.

### Expi293F cell transfection

See Examples 6 and 7 for more details of antibody expression and purification.

One day before transfection, cultured Expi293F cells (Invitrogen) were counted for cell seeding calculation. Expi293F cells were pelleted at 300 rpm for 10 minutes. Cells were resuspended in pre-warmed fresh Expi293 expression media to give a final dilution of 2.5×10⁶ cells/ml. 200 ml of cell suspension was incubated in a Kuhner Shaking Incubator (37°C, 140 rpm, 8% CO₂) until next morning.

On the day of transfection, cultured Expi293F cells were counted and diluted to 4×10⁶ cells/ml using fresh Expi293 expression media. 500 µl of cell suspension was aliquoted into each well of 96-well deep well plates using Multidrop Combi. After dispensing, the plates were covered with Duetz sandwich cover, and incubated in a Kuhner Shaking Incubator (37°C, 300 rpm, 50 mm orbital throw, humidity 80%) for 2-2.5 hours.

For each transfection, two mixtures were prepared.
Mix 1: 25µl B cell bridge product + 55µl RSM + 100ng PBase per well
Mix 2: 1µl ExpiFectamine^{™} 293 (Invitrogen) + 79µl RSM

Mix 2 was added to Mix 1 and incubated at room temperature for 15 minutes. The incubated mixture was then added to the 500 µl of cell culture solution and incubated in a Kuhner Shaking Incubator (37°C, 300 rpm, 50mm orbital throw) for a week.

### Example 6: Expression of Antibodies

Mammalian transient expression systems enable flexible and rapid production of proteins. They are ideal for expression of human or other mammalian proteins because these systems generate recombinant proteins with more native folding and post-translational modifications.
1. The day before transfection, Expi293 cells were counted by an automated cell counter (Eve cell counter) seeded in pre-warmed expression media at the density of ~1.7×10⁶cells/ml and incubated overnight in an orbital shaker incubator (37°C, 8% CO2, 140 rpm)
2. On the day of transfection, cells were counted and adjusted the cell number to 2.5×10⁶cells/ml. 2000µl of cell suspension were dispensed into 24 Deep Well Plates and placed into Kuhner shaking incubator (37°C, 8% CO2, 225rpm)

### Preparation of DNA solutions

Mixed solutions containing plasmid DNA were prepared for transfection of Expi293 cells. For 2 mL of cell culture, 1 µg each ofthe heavy chain expression plasmid and the light chain expression plasmid were used. 2µg of DNA plasmid mixture diluted in ultrapure water was used for each transfection.

### Transfection

Two mixtures were prepared:
Mix 1: 2µg of plasmid DNA diluted in 40µl of Opti-MEM^{®} I medium.
Mix 2: 80µl of ExpiFectamine^{™} 293 transfection reagent + 40µl of OptiMEM I medium.

Mix 2 was combined with Mix 1 and incubated for 20-30 minutes at room temperature. After the incubation was complete 165µl of DNA-ExpiFectamine^{™} 293 Reagent complex was dispensed to each well of the 24 deep well plate. Cells were incubated in the Kuhner shaking incubator (37°C, 8% CO2, 225rpm) for 6 days. Cell culture supernatants were harvested 6 days after transfection.

### Example 7: Purification of Antibodies from Common Light Chain Mouse

A 96-well plate purification was employed to purify antibodies from Expi293F cell culture supernatants. This method allows rapid small-scale affinity antibody purification of multiple samples in antibody screening experiments. To achieve high % of recovery MabSelect Sure LX^{™} was used which is a protein A affinity with very dynamic binding activity (60 mg human IgG/ml medium), an extended residence time, alkali tolerant and low ligand leakage.

### Procedure

1. MabSelect Sure LX resin (GE Healthcare) was equilibrated in 1X PBS (Gibco) to remove the storage buffer, to a final concentration of 10 % slurry.
2. 600 µl of 10 % slurry is added to each well of the 96 well plate AcroPrep^{™} Advance (Pall)
3. The resin was centrifuged 70 x rcf for 1 min at 4 C.
4. The resin was washed with 300 µl 1X PBS, spin 70x rcf for 1 min. This step was repeated twice.
5. 2 ml of cell culture supernatants (pH 7-8) were loaded onto the MabSelect Sure LX resin into the 96 well purification plate and centrifuged at 70 - 100 x rcf for 1 minute.
6. Plate was washed using 600 µl of 1 x PBS and centrifuged at 70 - 100 x rcf for 1 minute. This step is performed four times in total.
7. 70 µl of elution buffer (IgG Elute Pierce) was added to each well and incubated for 1 min.
8. The plate was centrifuged at 70 - 100 x rcf for 1 minute to collect the eluate. This step is performed two times in total.

### Example 8: SPR Analysis of Antibody for Antigen Y

### AIM of experiment

A SPR analysis was performed to determine the binding affinity and the kinetics of interaction of the Hit 1 (ie, a lambda-type antibody from our common light chain mouse) to Target Y. The affinity and the kinetics of the purified antibody was compared to the benchmark and an isotype control (ISTC).

### Methods

Surface plasmon resonance (SPR) was used to determine the binding affinity (K_{D}) to the antigen Y, the kinetic constants on-rate (kₒₙ) and off-rate (k_{off}). The analysis was performed using a Biacore 8K (GE Healthcare) system.

An anti-human IgG Fc antibody was immobilised on CM4 chip (GE Healthcare catalogue number BR100534) according to manufacturer's instructions. Amine coupling kit (BioRad) was used to activate the surface of the chip. The surface was subsequently blocked with 1M ethanolamine. The immobilisation run was performed at 25°C using HBS-EP as immobilisation running buffer.

The purified monospecific antibodies (referred as ligand) from Example 7 were captured onto the anti-human IgG Fc CM4 surface at approximately 2ug/ml. The ligands were injected for 60 seconds at 10 ul/min in all the active channels of all 8 flow channels. The run was performed at 25°C using neutral pH HBS-P 1X + CaCl2 2.5 mM as running buffer.

Human antigen Y was reconstituted at 1mg/ml in the running buffer and used as analyte. The antigen Y was injected in multiple cycle kinetics (MCK) mode at 3 concentrations (1.5uM, 500nM and 166.7 nM) with 120 seconds association phase and 200 seconds dissociation phase, at flow rate 30ul/sec in both active and reference channels. Three injections of 10 mM Glycine pH 1.5 for 60 sec. at 10 µl/min were used for the regeneration phase.

An isotype control antibody hlgG4PE KYAB2229 (ISTC; this does not specifically bind antigen Y) was captured at 1ug/ml for 60 sec at 10 µl/min in the reference channel. An isotype control antibody hlgG4PE was also captured in the active channel as a negative control. Two antibodies against antigen Y, were used as benchmarks (Benchmark 1 and Benchmark 2). The data were reference and buffer subtracted and fitted into Langmuir 1:1 model. The first 30 seconds of dissociation were evaluated in the model.

**Table 2. Binding affinity and kinetic constants on-rate (kₒₙ) and off-rate (k_{off}) of anti-antigen Y antibodies**

| **Captured Antibody** | **kₒₙ (1/Ms)** | **k_{off} (1/s)** | **K_{D} (M)** |
|---|---|---|---|
| Benchmark 1 | 2.94 × 10⁴ | 4.27 × 10 ⁻² | 1.45 × 10 ⁻⁶ |
| Benchmark 2 | 4.13 × 10⁴ | 2.72 × 10 ⁻² | 6.60 × 10 ⁻⁷ |
| Antibody Hit 1 | 1.19 × 10⁵ | 1.25 × 10 ⁻¹ | 1.05 × 10 ⁻⁶ |
| hlgG4PE ISTC | | | NB |

The association and dissociation data of the interaction were fitted using biomolecular reaction model (1:1 Langmuir model). The values for association rate constant (kₒₙ), dissociation rate constant (k_{off}) and dissociation constant (K_{D)} were calculated from the binding data by BIAevaluation software.
Benchmark 1: antibody anti-antigen Y
Benchmark 2: antibody anti-antigen Y
NB: no binding observed
hlgG4PE ISTC: human isotype control IgG4

Hit 1 antibody was in IgG4PE format.

### Conclusion

Hit 1 showed binding to antigen Y in the affinity range provided in Table 2 and fast association (kₒₙ) and dissociation (k_{off}) rates for antigen Y. No binding to antigen Y was observed with ISTC.

Hit 1 antibody shows similar kinetic rates and binding affinity to antigen Y compared to the benchmark antibodies. This shows that our common light chain mice can generate antibodies with human variable domains that specifically bind to target antigen with desirable binding kinetics.

### Example 9: Analysis of B-Cell Populations from Common Light Chain Mice

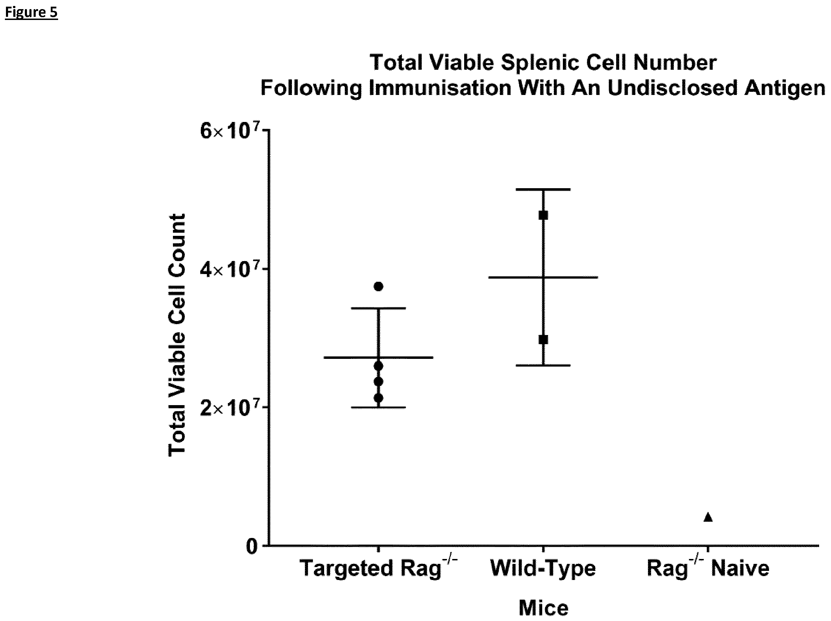

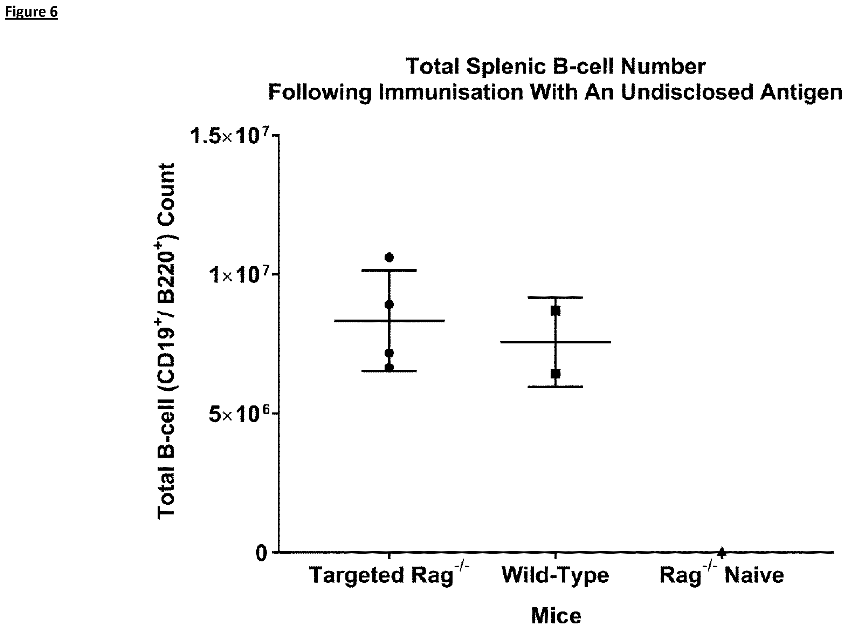

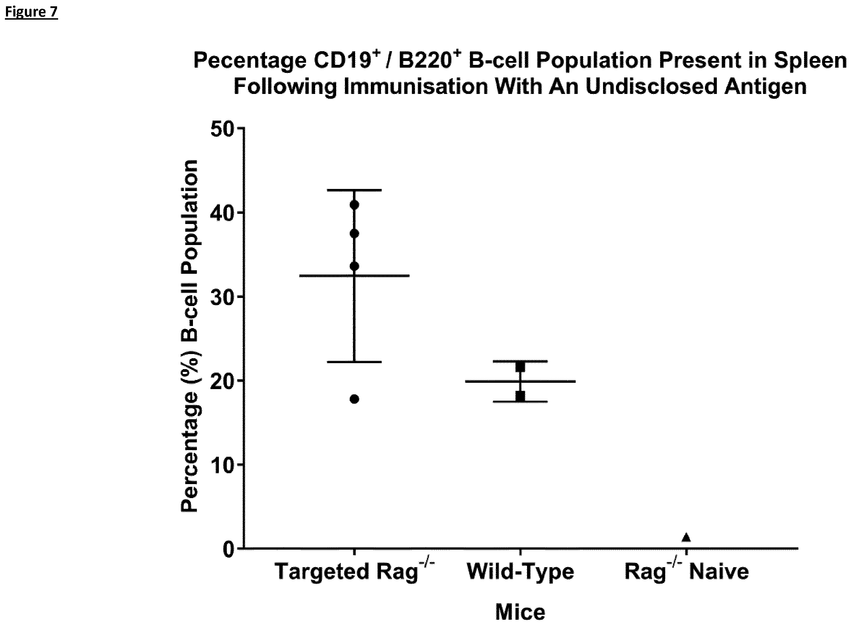

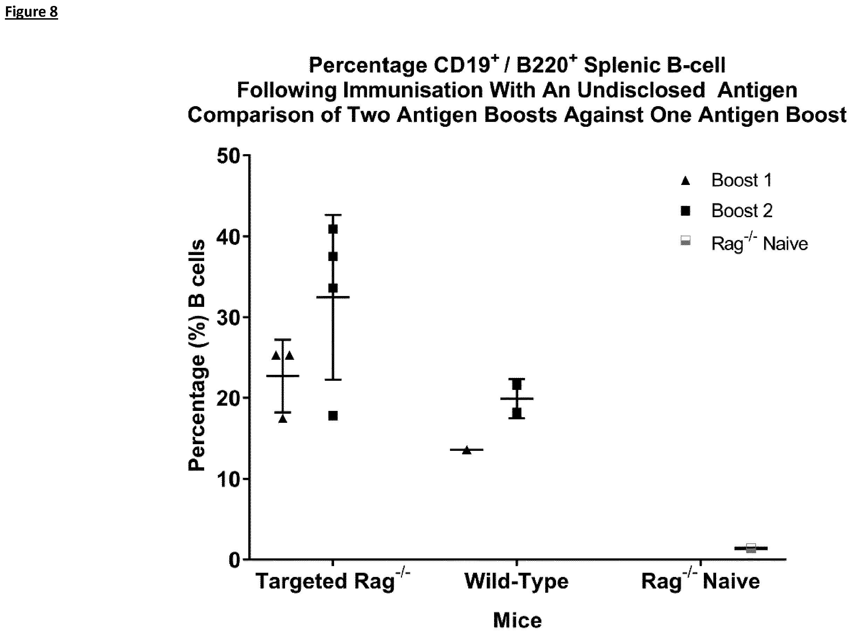

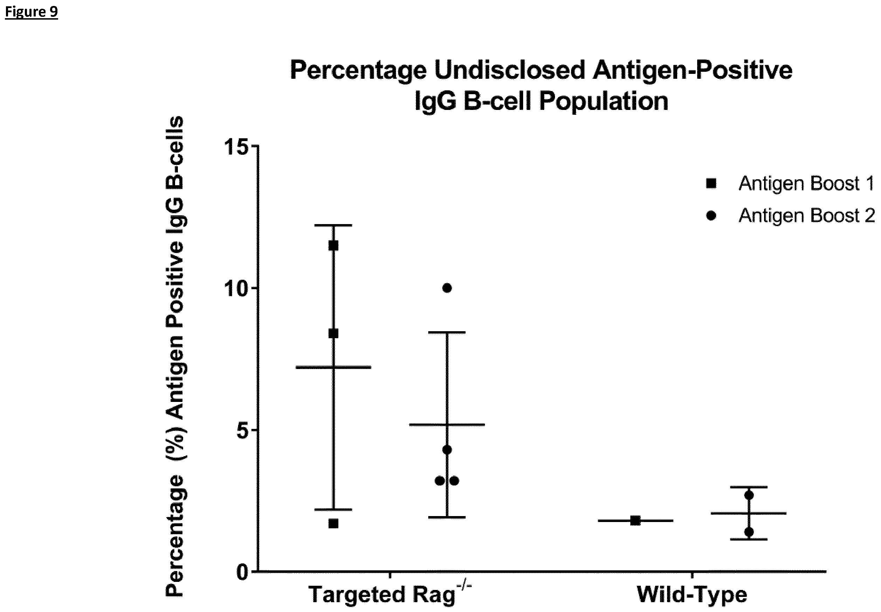

We analysed the B-cell compartments of mice of the invention. The results are shown in figures 5 to 9 as follows. "Targeted Rag^{-/-}" mice were mice produced as described in Example 4, derived from engineered ES cells. Thus, the genomes of the mice comprised the rearranged human lambda light chain knock-in and an unrearranged human heavy chain variable region comprised by a IgH locus.

### Figure 5

Chimera mice generated from Rag^{-/-} blastocysts micro-injected with targeted ES cells (targeted Rag^{-/-}) had similar total viable spleen cell number compared to wild-type mice (Wild-Type). Total viable spleen cell number following immunisation (antigen prime followed by two antigen boosts) with undisclosed antigen (Target Y). As a control, un-immunised Rag^{-/-} deficient mice displayed low total viable spleen cell number. Targeted Rag^{-/-}, chimera mouse derived from targeted ES-cells and Rag^{-/-}deficient blastocysts; Wild-Type, wild-type mouse; Rag^{-/-} Naive, un-immunised Rag^{-/-} deficient mice.

### Figure 6

Chimera mice generated from Rag^{-/-} blastocysts micro-injected with targeted ES cells (targeted Rag^{-/-}) had similar CD19-postive, B220-positve B-cell number compared to wild-type mice (Wild-Type). Total splenic B-cell number following immunisation (antigen prime followed by two antigen boosts) with undisclosed antigen (Target Y). As a control, un-immunised Rag^{-/-} deficient mice (Rag^{-/-} Naive) displayed minimal total B-cell (CD19⁺ / B220⁺) number. Targeted Rag^{-/-,} chimera mouse derived from targeted ES-cells and Rag^{-/-} deficient blastocysts; Wild-Type, wild-type mouse; Rag^{-/-} Naive, un-immunised Rag^{-/-} deficient mice.

### Figure 7

Percentage CD19-positive, B220-positive splenic B-cell population in chimera mice generated from Rag^{-/-} blastocysts micro-injected with targeted ES cells (targeted Rag^{-/-}) was similar to wild-type mice (Wild-Type). Percentage CD19-positive, B220-positive splenic B-cell population following immunisation (antigen prime followed by two antigen boosts) with undisclosed antigen (Target Y). As a control, un-immunised Rag^{-/-} deficient mice (Rag^{-/-} Naive) displayed minimal percentage CD19-positive, B220-positive splenic B-cell population. Targeted Rag^{-/-}, chimera mouse derived from targeted ES-cells and Rag^{-/-} deficient blastocysts; Wild-Type, wild-type mouse; Rag^{-/-} Naive, un-immunised Rag^{-/-} deficient mice.

### Figure 8

Percentage CD19-positive, B220-positive splenic B-cell population in chimera mice generated from Rag^{-/-} blastocysts micro-injected with targeted ES cells (targeted Rag^{-/-}) was similar to wild-type mice (Wild-Type). Percentage CD19-positive, B220-positive splenic B-cell population following immunisation (antigen prime followed by either one or two antigen boosts) with undisclosed antigen (Target Y). As a control, un-immunised Rag^{-/-} deficient mice (Rag^{-/-} Naive) displayed minimal percentage CD19-positive, B220-positive splenic B-cell population. Targeted Rag^{-/-}, chimera mouse derived from targeted engineered ES-cells and Rag^{-/-} deficient blastocysts; Wild-Type, wild-type mouse; Rag^{-/-} Naive, un-immunised Rag^{-/-} deficient mice.

### Figure 9

Percentage antigen positive IgG B-cell population in chimera mice generated from Rag^{-/-} blastocysts micro-injected with targeted ES cells (targeted Rag^{-/-}) was similar to wild-type mice (Wild-Type). Percentage antigen positive, CD19-positive or B220-positive, IgM-negative and IgD-negative splenic B-cell population following immunisation (antigen prime followed by either one or two antigen boosts) with undisclosed antigen (Target Y). Targeted Rag^{-/-}, chimera mouse derived from targeted engineered ES-cells and Rag^{-/-} deficient mice; Wild-Type, wild-type mouse.

### Conclusion

"Targeted Rag^{-/-}" chimera mice derived from engineered ES cells micro-injected into Rag^{-/-} deficient blastocysts produced IgM-negative, IgD-negative, antigen-positive splenic B-cells following an antigen prime and either one or two antigen boosts in a manner similar to wild-type mice. In comparison, non-chimeric Rag^{-/-} deficient mice did not produce splenic B-cells. These observations support our observation that mice generated from engineered ES cells micro-injected into Rag^{-/-}blastocysts have a functional immune response which generated antigen positive IgG B-cells in response to an immunisation regime.

Usefully, a respective copy of the common light chain can be paired with a copy of a heavy chain from an antibody (isolated from an immunised common light chain mouse of the invention) that binds to Target Y and a copy of a heavy chain from an antibody that binds Target Z to produce a bispecific 4-chain antibody that specifically binds Y and Z. This can be expressed from a cell (eg, a eukaryotic, mammalian, human, CHO, Cos or HEK293 cell) that comprises (i) an expressible nucleotide sequence encoding the light chain; (ii) an expressible nucleotide sequence encoding a heavy chain that specifically binds Target Y; and (iii) an expressible nucleotide sequence encoding a heavy chain that specifically binds Target Z. Alternatively, the cell comprises (i) and (ii) and a second cell comprises (iii), wherein the heavy and light chain expression products of the cells can be mixed to produce the bispecific antibody. Alternatively, the cell comprises (i) and (iii) and a second cell comprises (ii), wherein the heavy and light chain expression products of the cells can be mixed to produce the bispecific antibody. Alternatively, the cell comprises (ii) and (iii) and a second cell comprises (i), wherein the heavy and light chain expression products of the cells can be mixed to produce the bispecific antibody. Alternatively, (i), (ii) and (iii) are comprised by separate, respective cells and the antibody chain expression products of the cells are mixed to produce a bispecific antibody. In an example, the heavy chain constant regions comprise motifs or mutations (eg, charge pairs or knobs-in-holes motifs, see references above) that promote pairing of the anti-Target Y heavy chain with the anti-Target Z heavy chain.

### Methodology

### Introduction:

Following immunisation with antigen Y, splenic B-cells expressing class switched antibodies specific for said antigen were processed and single cell sorted. Targeted Rag^{-/-} chimera mice were analysed for evidence of a functional immune system and were compared to wild-type control mice. An additional comparison was also made to non-chimeric Rag^{-/-} mice, which demonstrate a minimal baseline immune response.

### Materials :

**Table 3: Table of reagents:**

| **Reagent** | **Supplier** | **Cat #** | **Lot #** |
|---|---|---|---|
| RPMI | Gibco | 11835-063 | 1881906 |
| Heat-inactivated Foetal Bovine Serum | Gibco | 10270-106 | 08G2073K |
| RNA Lysis buffer | Epicentre | QER090150 | 20151690 |
| TruStain fcX/Fc blocker | Biolegend | 101320 | B221805 |
| 7-AAD | eBioscience | 00-6993-50 | 4298663 |
| CD19-BUV395 | BD Horizon | 563557 | 7125716 |
| CD45R/B220-BUV395 | BD Horizon | 563793 | 7177756 |
| IgM-BV786 | BD Horizon | 564028 | 7048650 |
| IgD-BV605 | BD Horizon | 563003 | 7226860 |
| Ly-6G-APCCy7 | BD Pharmingen | 557661 | 7040615 |
| CD8a-APC-H7 | BD Pharmingen | 560182 | 7110593 |
| F4/80-APCCy7 | Biolegend | 123118 | B217243 |
| CD4-APC-H7 | BD Pharmingen | 560181 | 7012678 |
| CD11c-APC-Cy7 | BD Pharmingen | 561241 | 7096608 |
| ACK Lysis Buffer | Life Technologies | A10492-01 | N/A |
| Undisclosed antigen Y-647 (1 µg/ml) | In-house | | |

### Method:

### Processing of samples:

Spleens from immunised mice were removed and homogenised using 40 µm cell strainers using aseptic procedures. Cells were pelleted by centrifugation (350 rcf for 10 minutes at 4 °C) and the supernatant was carefully aspirated. Red blood cells were removed by lysis using 1 ml of ACK lysis buffer and incubated for 2 minutes at room-temperature. The lysis reaction was terminated by adding an excess of 3 % FBS / RPMI buffer. Cells were subsequently filtered using 40 µm cell strainers, pelleted as before and resuspended in 340 µl buffer. Any Fc receptors expressed on processed cells were blocked, removing non-specific staining of B-cells whilst maintaining antibody-mediated specific staining, using Fc blocker. At this stage a total cell count was taken.

### Staining:

Single-cell suspensions prepared from spleens were stained in the following manner. Mature, class-switched B-cell spleen populations were defined using BUV395-conjugated anti-CD45R (selecting), BUV395-conjugated anti-CD19 (selecting), BV786-conjugated anti-IgM (depleting) and BV605-conjugated anti-IgD (depleting). Cells other than B-cell such as neutrophils and monocytes, CD8-postive T-cells, macrophages, CD4 positive T-cells and dendritic cells were excluded using APCCy7-conjugated anti-Ly-6G/C, APC-H7-conjugated anti-CD8a, APCCy7-conjugated anti-F4/80, APC-H7-conjugated anti-CD4 and APC-Cy7-conjugated anti-CD11c, respectively. Antigen positive populations were identified using the same antigen used for immunisation labelled with Alexa-647. Non-viable cells were excluded using 7-AAD. An antigen-unlabelled control pool of cells was created to allow a FMO (Fluorescence Minus One) to be derived. Additional positive staining controls and 7AAD (live/dead) controls were also taken. All staining reactions were incubated at 4 °C for 20 min using 1 × 10⁶ cells in 100 µl of PBS with 3% FBS. Samples were washed twice in 400 µl PBS with 3% FBS and analyzed on BD FACSAria Fusion flow cytometer (BD Biosciences). All acquired data were analyzed using Flow-Jo^{™} software. Cells of interest were single cell sorted directly into 96 well plates containing RNA lysis buffer. Samples were then processed using B-Cell Technology (BCT).

**Aspects of the invention are disclosed in the following numbered clauses::**
1. A cell comprising an antibody light chain locus (first locus), wherein the locus comprises (in 5' to 3' direction)
   (a) a light chain intronic enhancer;
   (b) a rearranged antibody variable region encoding a rearranged antibody V domain; and
   (c) an antibody light chain constant region encoding a light chain C domain;
   wherein the locus is operable to express an antibody chain comprising (in N- to C-terminal direction) said rearranged V domain and said C domain.
2. The cell of clause 1, wherein the locus is a kappa light chain locus and the enhancer is Eiκ.
3. The cell of clause 1, wherein the cell is a mouse cell, locus is a lambda light chain locus and the enhancer is a mouse lambda 2-4 or 4-10 enhancer.
4. The cell of any preceding clause, wherein
   (a) the rearranged V domain is a Yκ and the C domain is a Cκ;
   (b) the rearranged V domain is a Yκ and the C domain is a Cλ;
   (c) the rearranged V domain is a Vλ and the C domain is a Cλ; or
   (d) the rearranged V domain is a Vλ and the C domain is a Cκ.
5. A cell comprising an antibody heavy chain locus (first locus), wherein the locus comprises (in 5' to 3' direction)
   (a) a heavy chain intronic enhancer (Eµ);
   (b) a rearranged antibody variable region encoding a rearranged antibody V domain;
   (c) an optional switch sequence; and
   (d) an antibody heavy chain constant region encoding a heavy chain CH1 domain;
   wherein the locus is operable to express an antibody chain comprising (in N- to C-terminal direction) said rearranged V domain and said CH1 domain.
6. The cell of any preceding clause, wherein the locus comprises (in 5' to 3' direction)
   (a) a promoter operable for promoting transcription of the variable region;
   (b) a nucleotide sequence encoding a variable domain signal peptide;
   (c) said intronic enhancer;
   (d) said rearranged antibody variable region; and
   (e) said antibody light chain constant region,
   Wherein the locus is operable to express RNA transcripts encoding (in N- to C-terminal direction) said signal peptide sequence fused to the amino acid sequence of said variable domain.
7. The cell of any preceding clause, wherein the cell is an ES, iPS, hybridoma or B-cell.
8. The cell of any preceding clause, wherein the rearranged V domain is a kappa or lambda V domain.
9. The cell of any preceding clause, wherein the variable region encodes for a V domain that has a binding specificity for a first predetermined antigen or a first epitope, wherein the locus is operable to express an antibody chain that comprises a V domain that retains said specificity.
10. The cell of any preceding clause, wherein the cell comprises a second antibody locus that is operable to express a second antibody chain, wherein the second chain comprises a second rearranged V domain that forms a binding site with the first rearranged V domain, wherein the binding site is capable of specifically binding to a predetermined antigen or epitope.
11. The cell of clause 10 when dependent from clause 9, wherein the predetermined antigens are different and said other and second V domains are different; or wherein the epitopes are different and said other and second V domains are different.
12. The cell of clause 11, wherein
   (a) the first domain is a VL domain and the second and said other V domains are VH domains; or
   (b) wherein the first domain is a VH domain and the second and said other V domains are VL domains.
13. The cell of any preceding clause, wherein the variable region is within 0.5 kb 3' of the enhancer.
14. The cell of any preceding clause, wherein the locus comprises a second enhancer that is 3' of the constant region.
15. The cell of any preceding clause, wherein the cell is a non-human mammal, mouse, rat or rodent cell.
16. The cell of any preceding clause, wherein said constant region is at an endogenous antibody locus of the cell.
17. The cell of clause 16 when dependent from clause 1, wherein said endogenous locus is an endogenous kappa chain locus.
18. The cell of clause 16 when dependent from clause 5, wherein said endogenous locus is an endogenous heavy chain locus.
19. The cell of any one of clauses 1 to 15, wherein the locus is comprised by a transgene that is comprised by the genome of the cell at a position outside an endogenous antibody locus.
20. The cell of any preceding clause, wherein the cell is homozygous for said locus.
21. The cell of any preceding clause when dependent from clause 1, wherein the cell genome comprises a second antibody locus, wherein the second locus is an unrearranged antibody heavy chain locus comprising (in 5' to 3' direction)
   (a) one or more VH gene segments;
   (b) one or more DH gene segments;
   (c) one or more JH gene segments; and
   (d) a heavy chain constant region encoding one or more CH domains;
   Wherein the heavy chain locus is operable to express a plurality of heavy chains, optionally comprising a plurality of antigen specificities or affinities, each said heavy chain being capable of pairing with an antibody chain encoded by the first locus to produce paired chains that comprise an antigen binding site.
22. The cell of any one of clauses 1 to 20 when dependent from clause 5, wherein the cell comprises one or more further antibody loci, each further locus being capable of expressing a light chain, each said light chain being capable of pairing with an antibody chain encoded by the first locus to produce paired chains that comprise an antigen binding site.
23. The cell of any preceding clause, wherein each variable region or gene segment is human.
24. The cell of any preceding clause, wherein the cell is a mouse cell and each constant region is a mouse, rat or human constant region.
25. The cell of any preceding clause, wherein each said enhancer is an endogenous enhancer of the cell.
26. The cell of any preceding clause, wherein the cell is a mouse cell and each said enhancer is a mouse enhancer.
27. The cell of any preceding clause, wherein
   (a) the cell is a mouse cell, the variable region is a human variable region, the intronic enhancer is a mouse intronic enhancer at an endogenous antibody locus of the cell and said constant region is a mouse, rat or human constant region; or
   (b) the cell is a rat cell, the variable region is a human variable region, the intronic enhancer is a rat intronic enhancer at an endogenous antibody locus of the cell and said constant region is a mouse, rat or human constant region.
28. The cell of any preceding clause, wherein the rearranged variable region is a rearrangement of
   (a) human IGLV3-21 and IGLJ3 and optionally operably connected to human germline IGLV3-21 promoter and/or signal peptide-encoding nucleotide sequence;
   (b) human IGK1-39 and IGKJ1 or 5 and optionally operably connected to human germline IGKV1-39 promoter and/or signal peptide-encoding nucleotide sequence;
   (c) human IGK3-20 and IGKJ1 or 5 and optionally operably connected to human germline IGKV3-20 promoter and/or signal peptide-encoding nucleotide sequence;
   (d) a human VpreB and Jλ5 and optionally operably connected to human germline VpreB promoter and/or signal peptide-encoding nucleotide sequence.
29. A transgenic non-human vertebrate comprising a plurality of cells according to any preceding clause.
30. The vertebrate of clause 29, wherein the germline of said vertebrate comprises a first locus (and optionally the second locus) as defined in any one of clauses 1 to 28.
31. The vertebrate of clause 29, wherein the vertebrate is a chimaera of said cells and a plurality of other cells that do not comprise the first locus, wherein the germline of the vertebrate does not comprise a said first locus.
32. The vertebrate of any one of clauses 29 to 31, comprising a first plurality of cells and a second plurality of cells, wherein the cells of the first plurality comprise the same first identical antibody locus, and the cells of the second plurality comprise the same additional identical antibody locus, wherein each said antibody locus is according to the first locus of any one of Statements 1 to 28, wherein the vertebrate is capable of expressing a first plurality of antibody chains from said first identical locus and a second plurality of antibody chains from said additional locus, and wherein the antibody locus of the first cells is different from the antibody locus of the second cells.
33. The vertebrate of clause 32, wherein the vertebrate comprises a third plurality of cells, wherein the cells of the third plurality comprise the same further antibody locus, wherein the further locus is according to any one of clauses 1 to 28 and is different from said antibody loci of the first and second cells, wherein the vertebrate is capable of expressing a third plurality of antibody chains from the further locus.
34. The vertebrate of clause 32 or 33, wherein the vertebrate is a chimaera of said cells and a plurality of other cells that do not comprise a first locus according to any one of clauses 1 to 28 or said additional or further loci.
35. A transgenic non-human vertebrate whose antibody light chain repertoire is at least 95% pure for a single rearranged VL domain species.
36. The vertebrate of clause 35, wherein the repertoire is at least 99% pure for a single rearranged VL domain species.
37. The vertebrate of clause 35 or 36, wherein the remainder of the light chains of the light chain repertoire comprise mutants of said VL domain species.
38. A transgenic non-human vertebrate wherein at least 95% of all VL domains of the antibody light chain repertoire of the vertebrate are encoded by the same light chain variable region sequence comprised by the genome of the vertebrate.
39. The vertebrate of clause 38, wherein at least 99% of all VL domains derived from said recombination comprise the same VL amino acid sequence.
40. A plurality of spleen, bone marrow, B-cells or blood cells of a vertebrate according to any one of clauses 29 to 39 comprising a plurality of first loci according to any one of clauses 1 to 28.
41. A population of spleen, bone marrow, B-cells, hybridomas, CHO cells, HEK cells, MEF cells, COS cells, HeLa cells or blood cells, wherein each cell is according to clause 1 or any one of clauses 2 to 28 when dependent from clause 1; and optionally, wherein the cells express at least 10 different antibody species wherein
   (a) at least 95% of the antibodies share the same light chain VL domain and the population comprises at least 10 different VH domain species; or
   (b) the antibodies comprise VL domains derived from the recombination of a first VL gene segment and a first JL gene segment, wherein at least 95% of all said VL domains derived from said recombination comprise the same VL amino acid sequence and the population comprises at least 10 different VH domain species.
42. A polyclonal antibody population, wherein at least 95% of the antibodies comprised by the population share the same light chain VL domain and the population comprises at least 10 different VH domain species.
43. A polyclonal antibody population, comprising VL domains derived from the recombination of a first VL gene segment and a first JL gene segment, wherein at least 95% of all said VL domains derived from said recombination comprise the same VL amino acid sequence and the population comprises at least 10 different VH domain species.
44. A method for identifying or obtaining an antibody, an antibody variable domain, a nucleotide sequence encoding an antibody or an antibody variable domain, or an expression vector or host cell that is capable of expressing the antibody or domain, wherein the antibody or domain is capable of specifically binding to a target antigen, the method comprising
   (a) contacting the cell population of clause 41, or the antibody population of 42 or 43 with said antigen;
   (b) binding antibodies expressed or comprised by said population to said antigen; and
   (c) isolating or identifying one or more antibodies that bind to the antigen, or isolating or identifying a VH and/or a VL domain of a said one or more antibody; or identifying a nucleotide sequence encoding a said VH or VL; and
   (d) optionally
      (i) correlating a said identified antibody or domain with a nucleotide sequence encoding therefor, thereby identifying said sequence;
      (ii) amplifying said sequence and inserting the sequence into an expression vector or a host cell genome for expression of the encoded antibody or domain; and
      (iii) optionally expressing and isolating said antibody or domain,
      wherein steps (i) and (ii) can be carried out in any order.
45. The method of clause 44, comprising using the method to amplify the VH and VL nucleotide sequences encoding the VH and VL domains of the identified antibody and further comprising inserting the VH and VL nucleotide sequences into the vector or cell for co-expression of the VH and VL to produce paired VH/VL binding sites that are capable of binding to the antigen, and expressing (and optionally isolating) the binding sites.
46. The method of clause 45, wherein the method comprises expressing the VH and VL in the presence of a further species of VH domain, wherein the VL and the further VH form further paired VH/VL sites that are capable of binding to a further antigen, wherein the further antigen and the antigen recited in clause 45 are different, the method comprising co-expressing (and optionally isolating) the binding sites wherein the binding sites are comprised by bi-specific antibodies encoded by the vector or cell, wherein the bi-specific antibodies comprise a respective binding site for each said antigen.
47. The method of clause 46, wherein the bi-specific antibodies are 4-chain antibodies comprising a first heavy chain-light chain pair and a second heavy chain-light chain pair, wherein
   (a) the light chain in the first and second pairs comprises said VL domain,
   (b) the heavy chain of the first pair comprises the VH domain recited in clause 45, wherein the VH domain forms a VH/VL binding site with said VL domain and wherein the binding site is capable of specifically binding to said antigen recited in clause 45;
   (c) the heavy chain of the second pair comprises said further VH domain that forms a VH/VL binding site with said VL domain and wherein the binding site is capable of specifically binding to said further antigen recited in clause 46.
48. The method of clause 47, wherein each 4-chain antibody comprises mutated heavy chain constant regions to promote pairing of the heavy chains of the first and second heavy-light pairs; optionally wherein the mutations are knob-in-hole mutations or charge pair mutations.
49. A plurality of B-cells, hybridomas, CHO cells, HEK cells, MEF cells, COS cells, HeLa cells that expresses the polyclonal antibody population of clause 42 or 43.
50. The method of any one of clauses 44 to 48, wherein the method comprises obtaining VL and/or VH-encoding nucleotide sequences from the cells of clause 41 or 47, and in step (d) (i) using the nucleotide sequences to correlate one or more thereof with one or more VH and/or VL domains of said antibodies that bind the antigen.
51. A method for obtaining a nucleotide sequence encoding an antibody or an antibody variable domain, wherein the antibody or domain is capable of specifically binding to a target antigen, the method comprising
   (a) obtaining VL and/or VH-encoding nucleotide sequences from the cells of clause 41 or 47;
   (b) amplifying said sequence; and
   (c) optionally inserting the sequence into an expression vector or a host cell genome for expression of the encoded antibody or domain.
52. A method for obtaining an antibody-producing cell line for the production of antibodies that specifically bind to an antigen, the method comprising inserting VH and VL-encoding nucleotide sequences into the genome of a host cell, wherein
   (a) each VH sequence is operably inserted 5' of an antibody heavy chain constant region for expression of heavy chains by the cell comprising VH and C domains;
   (b) each VL sequence is operably inserted 5' of an antibody light chain constant region for expression of light chains by the cell comprising VL and C domains;
   (c) wherein the expressed heavy chains are capable of pairing with the light chains to produce heavy-light chain pairs, each pair comprising a VH/VL binding site that is capable of binding to an antigen; and
   (d) wherein VH and VL-encoding nucleotide sequences are the sequences of VH and VL-encoding nucleotide sequences of one or more cells of clause 41 or 47.
53. The method of clause 52, comprising inserting first and second VH-encoding nucleotide sequences into the cell genome, wherein
   (a) the first VH (when expressed as part of first heavy chains) is capable of pairing with the VL (when expressed as part of light chains) to form a first VH/VL binding site that is capable of specifically binding to a first antigen;
   (b) the second VH (when expressed as part of second heavy chains) is capable of pairing with the VL (when expressed as part of light chains) to form a second VH/VL binding site that is capable of specifically binding to a second antigen; and
   (c) the first and second antigens are different;
      wherein the first and second heavy chains and the light chains are capable of pairing to produce bi-specific 4-chain antibodies comprising a first heavy chain-light chain pair and a second heavy chain-light chain pair, wherein
   (d) the heavy chain of the first heavy chain-light chain pair comprises the first VH; and
   (e) the heavy chain of the second heavy chain-light chain pair comprises the second VH domain.
54. A method for producing bi-specific 4- chain antibodies wherein the bi-specific antibodies comprise a respective binding site for first and second antigens, wherein the antigens are different, each antibody comprising a first heavy chain-light chain pair and a second heavy chain-light chain pair, wherein
   (a) the heavy chain of the first heavy chain-light chain pair comprises a first VH, wherein the first VH (when expressed as part of first heavy chains) is capable of pairing with a VL (when expressed as part of light chains) to form a first VH/VL binding site that is capable of specifically binding to the first antigen; and
   (b) the heavy chain of the second heavy chain-light chain pair comprises a second VH, wherein the second VH (when expressed as part of second heavy chains) is capable of pairing with said VL (when expressed as part of light chains) to form a second VH/VL binding site that is capable of specifically binding to the second antigen;
      the method comprising
   (c) producing a first cell line that is capable of expressing the first heavy chains by (i) inserting into a first cell genome a nucleotide sequence encoding said first VH, wherein the VH sequence is operably inserted 5' of an antibody heavy chain constant region for expression of first heavy chains comprising first VH and C domains; and (ii) culturing the cell to produce a first cell line that expresses first heavy chains;
   (d) producing a second cell line that is capable of expressing the second heavy chains by (i) inserting into a second cell genome a nucleotide sequence encoding said second VH, wherein the VH sequence is operably inserted 5' of an antibody heavy chain constant region for expression of second heavy chains comprising second VH and C domains; and (ii) culturing the cell to produce a second cell line that expresses second heavy chains;
   (e) expressing first heavy chains from the first cell line and second heavy chains from the second cell line and mixing together first heavy chains comprising said first VH, second heavy chains comprising said second VH and light chains comprising said VL, whereby (iii) first heavy chains pair with light chains to produce said first heavy chain-light chain pairs, (iv) second heavy chains pair with light chains to produce said second heavy chain-light chain pairs; and (v) the first heavy chain-light chain pairs pair with the second heavy chain-light chain pairs, thereby producing said bi-specific antibodies
   wherein VH and/or VL-encoding nucleotide sequences are the sequences of VH and/or VL-encoding nucleotide sequences of one or more cells of clause 41 or 47.
55. The method of clause 54, comprising
   (a) producing a third cell line that is capable of expressing the light chains by (i) inserting into a third cell genome a nucleotide sequence encoding said VL, wherein the VL sequence is operably inserted 5' of an antibody light chain constant region for expression of light chains comprising the VL and C domains; and (ii) culturing the cell to produce a third cell line that expresses the light chains;
   (b) expressing the light chains from the third cell line, wherein the light chains are the light chains of step (e) of clause 54.
56. The method of clause 54, comprising
   (a) inserting into the genome of the first and/or second cell or cell lines a nucleotide sequence encoding said VL, wherein the VL sequence is operably inserted 5' of an antibody light chain constant region for expression of light chains comprising the VL and C domains; and
   (b) expressing the light chains from the cell lines comprising the VH sequence, wherein the light chains are the light chains of step (e) of clause 54.
57. The method of clause 54, 55 or 56, wherein said mixing of chains is carried out by co-culturing the cell lines.
58. The method of clause 54, 55 or 56, wherein said mixing of chains is carried out by isolating chains expressed by the cell lines and mixing the isolated chains together.
59. A method for producing bi-specific 4-chain antibodies wherein the bi-specific antibodies comprise a respective binding site for first and second antigens, wherein the antigens are different, each antibody comprising a first heavy chain-light chain pair and a second heavy chain-light chain pair, wherein
   (a) the heavy chain of the first heavy chain-light chain pair comprises a first VH, wherein the first VH (when expressed as part of first heavy chains) is capable of pairing with a VL (when expressed as part of light chains) to form a first VH/VL binding site that is capable of specifically binding to the first antigen; and
   (b) the heavy chain of the second heavy chain-light chain pair comprises a second VH, wherein the second VH (when expressed as part of second heavy chains) is capable of pairing with said VL (when expressed as part of light chains) to form a second VH/VL binding site that is capable of specifically binding to the second antigen;
      the method comprising
   (c) mixing together first heavy chains comprising said first VH, second heavy chains comprising said second VH and light chains comprising said VL, whereby (i) first heavy chains pair with light chains to produce said first heavy chain-light chain pairs, (ii) second heavy chains pair with light chains to produce said second heavy chain-light chain pairs; and (iii) the first heavy chain-light chain pairs pair with the second heavy chain-light chain pairs, thereby producing said bi-specific antibodies
   wherein VH and/or VL are encoded by VH and/or VL-encoding nucleotide sequences, wherein the nucleotide sequences are the sequences of VH and/or VL-encoding nucleotide sequences of one or more cells of clause 41 or 47.
60. The method of any one of clauses 54 to 59, wherein each 4-chain antibody comprises mutated heavy chain constant regions to promote pairing of the heavy chains of the first and second heavy-light pairs; optionally wherein the mutations are knob-in-hole mutations or charge pair mutations.
61. A method for producing a pharmaceutical composition for treating or preventing a disease or condition in a human or non-human animal subject, the method comprising
   (a) expressing an antibody from a cell line produced by the method of clause 52 or 53; and
   (b) mixing the antibody with a diluent or excipient;
   (c) optionally wherein the antibody is identical to an antibody obtained by the method of any one of clauses 54 to 60.
62. A method for producing a pharmaceutical composition for treating or preventing a disease or condition in a human or non-human animal subject, the method comprising mixing a bi-specific antibody with a diluent or excipient, wherein the antibody is identical to an antibody obtained by the method of any one of clauses 54 to 60.
63. A method for producing a cell of any one of clauses 1 to 28 or vertebrate of any one of clauses 29 to 39, the method comprising
   (a) obtaining a nucleic acid comprising a rearranged antibody variable region encoding a rearranged antibody V domain; and
   (b) inserting the variable region or a copy thereof into the genome of a cell, whereby the variable region is comprised by an antibody locus in said genome,
      wherein the locus comprises (in 5' to 3' direction)
   (c) an antibody locus intronic enhancer;
   (d) said rearranged antibody variable region; and
   (e) an antibody constant region encoding an antibody C domain;
      wherein the locus is operable to express an antibody chain comprising (in N- to C-terminal direction) said rearranged V domain and said C domain; and
   (f) optionally, wherein the cell is a non-human vertebrate ES cell or an iPS cell, the method further comprising generating the non-human vertebrate of any one of clauses 29 to 39 from the cell.
64. The method of clause 63, wherein the variable domain is a VL and the C domain is CL.
65. The method of clause 63 or 64, wherein said inserting is carried out using homologous recombination or site-specific recombination with chromosomal DNA of the cell.
66. The method of any one of clauses 63 to 65,
   comprising obtaining a transgene comprising (in 5' to 3' direction)
   (a) said intronic enhancer;
   (b) said rearranged antibody variable region; and
   (c) said antibody constant region; and
   Inserting said transgene into the genome of the cell, whereby said antibody locus is comprised by the cell.
67. The method of any one of clauses 63 to 65, comprising inserting said variable region between
   (a) the intronic enhancer and the CL of a light chain locus of the cell genome; or
   (b) the intronic enhancer (Eµ) and the Cµ of a heavy chain locus of the cell genome.
68. The method of any one of clauses 63 to 67, wherein the variable region encodes a V domain of an antibody that specifically binds to a predetermined antigen.
69. The method of clause 68, wherein the step of obtaining the nucleic acid comprising the rearranged antibody variable region as recited in clause 63 comprises
   (a) obtaining a further cell that expresses said antibody recited in clause 68;
   (b) obtaining or copying
      i. a DNA sequence of the further cell that comprises a rearranged variable region sequence encoding the V domain; or
      ii. a RNA sequence of the further cell that comprises a rearranged variable region sequence encoding the V domain, and creating a DNA copy of said RNA sequence;
   thereby obtaining said nucleic acid.
70. The method of any one of clause 63 to 69, comprising culturing the resultant cell to produce a plurality of cells, wherein each cell is according to any one of clauses 1 to 28.
71. A non-human vertebrate blastocyst or pre-morula embryo implanted with an ES or iPS cell according to any one of clauses 1 to 28 or obtained according to the method of any one of clauses 63 to 70.
72. A nucleic acid comprising a transgene for use in the method of clause 66, the transgene comprising (in 5' to 3' direction)
   (a) said intronic enhancer;
   (b) said rearranged antibody variable region; and
   (c) said antibody constant region.
73. A nucleic acid comprising a rearranged variable region sequence encoding a rearranged V domain for use in the method of clause 67, the nucleic acid comprising said rearranged variable region sequence flanked
   (a) 5' by a homology arm for hybridising to a first nucleotide sequence of the cell genome, and/or
   (b) 3' by a homology arm for hybridising to a second nucleotide sequence of the cell genome;
      Wherein
   (c) the 5' homology arm is homologous to a sequence of an antibody light chain locus of the cell 5' of the CL region thereof, and the 3' homology arm is homologous to a sequence of said locus 3' of the intronic enhancer of the locus, whereby homologous recombination between the homology arms and the genome of the cell is capable of inserting the variable region sequence, thereby producing a locus comprising (in 5 to 3' direction) an intronic enhancer, said variable region and a CL region; or
   (d) the 5' homology arm is homologous to a sequence of a heavy chain antibody locus of the cell 5' of the Cµ region thereof, and the 3' homology arm is homologous to a sequence of said locus 3' of the intronic enhancer (Eµ) of the locus, whereby homologous recombination between the homology arms and the genome of the cell is capable of inserting the variable region sequence, thereby producing a locus comprising (in 5 to 3' direction) an intronic enhancer, said variable region and a Cµ region.
74. The nucleic acid of clause 73, wherein the nucleic acid or 5' homology arm comprises (in 5' to 3' direction)
   (a) a promoter operable for promoting transcription of the variable region;
   (b) a nucleotide sequence encoding a variable domain signal peptide; and
   (c) Optionally a said intronic enhancer sequence,
   Wherein when the variable region sequence is inserted into the cell genome, the variable region is inserted in operable connection 3' of the promoter and signal peptide-encoding sequence for expression of an antibody chain comprising (in N- to C-terminal direction) a signal sequence-V domain-C domain.

## Claims

1. A cell comprising a first antibody light chain locus wherein the locus comprises in 5' to 3' direction:
(a) a light chain intronic enhancer;
(b) a rearranged antibody variable region encoding a rearranged antibody V domain; and
(c) an antibody light chain constant region encoding a light chain C domain;
wherein the locus is operable to express an antibody chain comprising (in N- to C-terminal direction) said rearranged V domain and said C domain.

2. The cell of claim 1, wherein the first antibody light chain locus is a kappa light chain locus and the enhancer is Eiκ.

3. The cell of claim 1, wherein the cell is a mouse cell, the first antibody light chain locus is a lambda light chain locus and the enhancer is a mouse lambda 2-4 or 4-10 enhancer.

4. The cell of any preceding claim, wherein
(a) the rearranged V domain is a VK and the C domain is a CK;
(b) the rearranged V domain is a VK and the C domain is a Cλ;
(c) the rearranged V domain is a Vλ and the C domain is a Cλ; or
(d) the rearranged V domain is a Vλ and the C domain is a CK.

5. The cell of any preceding claim, wherein the locus comprises in 5' to 3' direction:
(a) a promoter operable for promoting transcription of the variable region;
(b) a nucleotide sequence encoding a variable domain signal peptide;
(c) said intronic enhancer;
(d) said rearranged antibody variable region; and
(e) said antibody light chain constant region,
wherein the locus is operable to express RNA transcripts encoding in N- to C-terminal direction said signal peptide sequence fused to the amino acid sequence of said variable domain.

6. The cell of any preceding claim, wherein i) the cell is an ES, iPS, hybridoma or B-cell and/or the rearranged V domain is a kappa or lambda V domain.

7. The cell of any preceding claim, wherein the variable region encodes for a V domain that has a binding specificity for a first predetermined antigen or a first epitope, wherein the locus is operable to express an antibody chain that comprises a V domain that retains said specificity.

8. The cell of any preceding claim, wherein the cell comprises a second antibody locus that is operable to express a second antibody chain, wherein the second chain comprises a second rearranged V domain that forms a binding site with the first rearranged V domain, wherein the binding site is capable of specifically binding to a predetermined antigen or epitope, optionally wherein the predetermined antigens are different and said other and second V domains are different; or wherein the epitopes are different and said other and second V domains are different.

9. The cell of claim 8, wherein
(a) the first domain is a VL domain and the second and said other V domains are VH domains; or
(b) the first domain is a VH domain and the second and said other V domains are VL domains.

10. The cell of any preceding claim, wherein i) the variable region is within 0.5 kb 3' of the enhancer, ii) the locus comprises a second enhancer that is 3' of the constant region, iii) the cell is a non-human mammal, mouse, rat or rodent cell, iv) said constant region is at an endogenous antibody locus of the cell, optionally an endogenous kappa chain locus, v) the locus is comprised by a transgene that is comprised by the genome of the cell at a position outside an endogenous antibody locus and/or vi) the cell is homozygous for said locus.

11. The cell of any preceding claim, wherein the cell genome comprises a second antibody locus, wherein the second locus is an unrearranged antibody heavy chain locus comprising in 5' to 3' direction:
(a) one or more VH gene segments;
(b) one or more DH gene segments;
(c) one or more JH gene segments; and
(d) a heavy chain constant region encoding one or more CH domains;
wherein the heavy chain locus is operable to express a plurality of heavy chains, optionally comprising a plurality of antigen specificities or affinities, each said heavy chain being capable of pairing with an antibody chain encoded by the first locus to produce paired chains that comprise an antigen binding site.

12. The cell of any preceding claim, wherein
(a) the cell is a mouse cell, the variable region is a human variable region, the intronic enhancer is a mouse intronic enhancer at an endogenous antibody locus of the cell and said constant region is a mouse, rat or human constant region; or
(b) the cell is a rat cell, the variable region is a human variable region, the intronic enhancer is a rat intronic enhancer at an endogenous antibody locus of the cell and said constant region is a mouse, rat or human constant region.

13. A transgenic non-human vertebrate comprising a plurality of cells according to any preceding claim.

14. A population of spleen, bone marrow, B-cells, hybridomas, CHO cells, HEK cells, MEF cells, COS cells, HeLa cells or blood cells, wherein each cell is according to any one of claims 1-12; and optionally, wherein the cells express at least 10 different antibody species wherein
(a) at least 95% of the antibodies share the same light chain VL domain and the population comprises at least 10 different VH domain species; or
(b) the antibodies comprise VL domains derived from the recombination of a first VL gene segment and a first JL gene segment, wherein at least 95% of all said VL domains derived from said recombination comprise the same VL amino acid sequence and the population comprises at least 10 different VH domain species.

15. A method for identifying or obtaining an antibody, an antibody variable domain, a nucleotide sequence encoding an antibody or an antibody variable domain, or an expression vector or host cell that is capable of expressing the antibody or domain, wherein the antibody or domain is capable of specifically binding to a target antigen, the method comprising
(a) contacting the cell population of claim 14 with said antigen;
(b) binding antibodies expressed or comprised by said population to said antigen; and
(c) isolating or identifying one or more antibodies that bind to the antigen, or isolating or identifying a VH and/or a VL domain of a said one or more antibody; or identifying a nucleotide sequence encoding a said VH or VL; and
(d) optionally
(i) correlating a said identified antibody or domain with a nucleotide sequence encoding therefor, thereby identifying said sequence;
(ii) amplifying said sequence and inserting the sequence into an expression vector or a host cell genome for expression of the encoded antibody or domain; and
(iii) optionally expressing and isolating said antibody or domain,
wherein steps (i) and (ii) can be carried out in any order.
